(19) Europäisches Patentamt
European Patent Office
Office européen des brevets

(11) Veröffentlichungsnummer: **0 630 946 A1**

(12) # EUROPÄISCHE PATENTANMELDUNG

(21) Anmeldenummer: **94109158.9**

(51) Int. Cl.[5]: **C09B 62/08**, C09B 62/507

(22) Anmeldetag: **15.06.94**

(30) Priorität: **21.06.93 DE 4320447**

(43) Veröffentlichungstag der Anmeldung: **28.12.94 Patentblatt 94/52**

(84) Benannte Vertragsstaaten: **BE CH DE FR GB IT LI**

(71) Anmelder: **HOECHST AKTIENGESELLSCHAFT**
**Brüningstrasse 50**
**D-65929 Frankfurt am Main (DE)**

(72) Erfinder: **Dannheim, Jörg, Dr.**
**Strubbergstrasse 32**
**D-60489 Frankfurt/Main (DE)**
Erfinder: **Reiher, Uwe, Dr.**
**Sachsenring 8**
**D-65719 Hofheim am Taunus (DE)**
Erfinder: **Russ, Werner Hubert, Dr.**
**Wingertstrasse 8a**
**D-65439 Flörsheim/Main (DE)**

(54) **Wasserlösliche faserreakfive Azofarbstoffe, Verfahren zu ihrer Herstellung und ihre Verwendung.**

(57) Es werden faserreaktive, sulfogruppenhaltige Mono-, Dis- und Polyazofarbstoffe, wie Trisazofarbstoffe, beschrieben, die eine faserreaktive Gruppierung der allgemeinen Formel

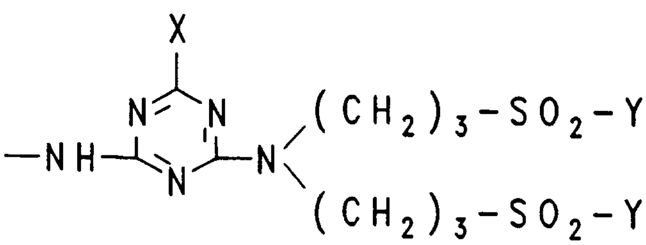

enthalten, in welcher X Chlor oder Fluor ist und die Gruppe $-SO_2-Y$ jedes eine faserreaktive Gruppe der Vinylsulfonreihe bedeutet, und die zusätzlich an anderer Stelle des Farbstoffmoleküls eine oder zwei weitere faserreaktive Gruppen der Vinylsulfonreihe besitzen. Sie eignen sich sehr gut zum Färben von hydroxy- und/oder carbonamidgruppenhaltigem Material, insbesondere Fasermaterial, wie Wolle und synthetischen Polyamidfasern und insbesondere Cellulosefasermaterialien, wie Baumwolle.

EP 0 630 946 A1

Die Erfindung liegt auf dem technischen Gebiet der faserreaktiven Farbstoffe.

Die Praxis des Färbens mit faserreaktiven Farbstoffen hat in neuerer Zeit zu erhöhten Anforderungen an die Qualität der Färbungen und die Wirtschaftlichkeit des Färbeprozesses geführt. Infolge dessen besteht weiterhin ein Bedarf nach neuen faserreaktiven Farbstoffen, die verbesserte Eigenschaften, nicht nur in Bezug auf die Echtheiten, sondern auch einen hohen Fixiergrad auf dem zu färbenden Material aufweisen. So sind aus den europäischen Patentanmeldungs-Veröffentlichungen Nrs. 0 070 806, 0 070 808 und 0 374 758, aus der britischen Patentschrift Nr. 1 576 237 und aus den US-Patentschriften Nrs. 4 908 436 und 5 138 041 faserreaktive Azofarbstoffe bekannt, die einen Monohalogen-s-triazinylamino-Rest enthalten, der mittels der Aminogruppe und einem aliphatischen Brückenglied mit einer faserreaktiven Gruppe der Vinylsulfonreihe verbunden ist. Diese bekannten Farbstoffe sind wegen der angesprochenen gestiegenen Anforderungen insbesondere hinsichtlich ihres Fixiergrades und der Farbstärke der mit ihnen erhältlichen Färbungen und Drucke verbesserungswürdig.

Mit der vorliegenden Erfindung wurden nunmehr neue faserreaktive Azofarbstoffe der nachstehend angegebenen und definierten allgemeinen Formel (1) gefunden, die sich gegenüber den bekannten Farbstoffen vorteilhaft unterscheiden und Färbungen und Drucke mit hoher Farbstärke liefern.

$(Y^o - SO_2)_a - F - Z$ (1)

In Formel (1) bedeuten:

- F ist der Rest eines sulfogruppenhaltigen Mono-, Dis- oder Polyazofarbstoffes, wie Trisazofarbstoffes;
- $Y^o$ ist Vinyl oder ist Ethyl, das in $\beta$-Stellung durch einen unter Einwirkung von Alkali bei Bildung der Vinylgruppe eliminierbaren Substituenten substituiert ist;
- Z ist eine Gruppe der allgemeinen Formel (2)

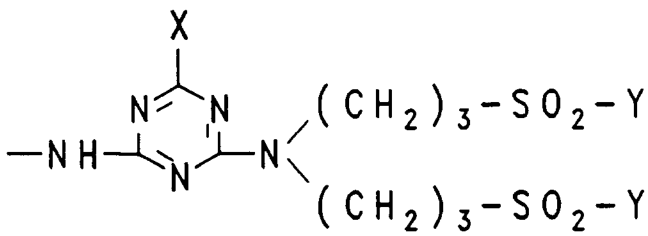

(2)

in welcher

- X Chlor oder Fluor ist und
- Y Vinyl ist oder Ethyl bedeutet, das in $\beta$-Stellung durch einen unter Einwirkung von Alkali bei Bildung der Vinylgruppe eliminierbaren Substituenten substituiert ist;
- a ist die Zahl 1 oder 2, bevorzugt 1.

Azofarbstoffe entsprechend der allgemeinen Formel (1) sind insbesondere solche, die den allgemeinen Formeln (1a) und (1b)

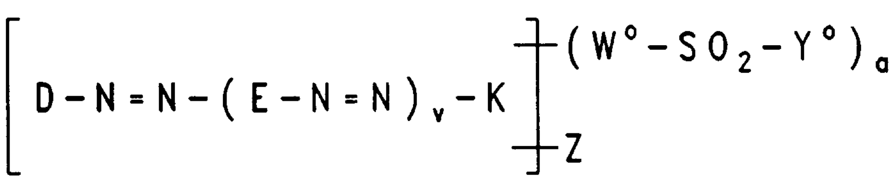

(1a)

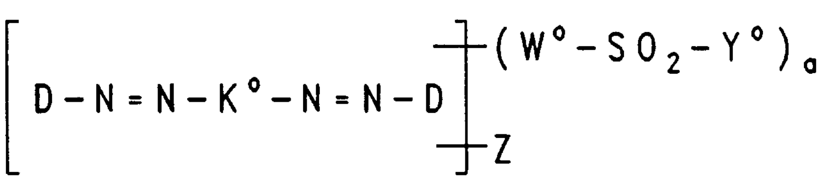

(1b)

entsprechen, in welcher bedeuten:

D
: ist als Rest einer Diazokomponente ein gegebenenfalls durch in Farbstoffen übliche Substituenten (wie beispielsweise durch Substituenten aus der später angegebenen Gruppe von Substituenten) substituierter Benzol- oder Naphthalinring, wobei D auch einen gegebenenfalls substituierten Phenylazo- oder Naphthylazo-Rest besitzen kann;

E
: ist als Rest einer diazotierbaren Kupplungskomponente ein gegebenenfalls durch in Farbstoffen übliche Substituenten (wie beispielsweise durch Substituenten aus der später angegebenen Gruppe von Substituenten) substituierter Benzol- oder Naphthalinring, der in ortho-Stellung zur Azogruppe eine Hydroxy- oder Aminogruppe besitzen kann;

K
: ist der Rest einer in Azofarbstoffen üblichen Kupplungskomponente, wie beispielsweise der Rest einer gegebenenfalls durch in Farbstoffen übliche Substituenten (wie aus der später angegebenen Gruppe von Substituenten) substituierten Kupplungskomponente der Benzol-, Naphthalin-, Pyrazolon-, 6-Hydroxypyridon(2)- oder Acetessigsäurearylamid-Reihe;

v
: ist die Zahl Null oder 1;

$K^o$
: ist der Rest einer bivalenten Kupplungskomponente, wie der bivalente Rest des gegebenenfalls durch in Farbstoffen übliche Substituenten substituierten Resorcins, 1,8-Dihydroxy-naphthalins oder 1-Amino-8-hydroxy-naphthalins;

$W^o$
: ist eine Alkylengruppe von 1 bis 4 C-Atomen, wie insbesondere die Methylen- oder Ethylengruppe, oder eine Gruppe der allgemeinen Formel $-NH-(C_2-C_4\text{-alkylen})-$ oder ist eine kovalente Bindung;

$Y^o$, a und Z
: haben eine der obengenannten Bedeutungen;

in Formel (1a) stehen die Gruppe(n) $-W^o-SO_2-Y^o$ an D und die Gruppe Z an K oder die Gruppe Z an D und die Gruppe(n) $-W^o-SO_2-Y^o$ an K gebunden, und

in Formel (1b) stehen die Gruppe(n) $-W^o-SO_2-Y^o$ an das eine D und die Gruppe Z an das andere D gebunden.

Alkalisch eliminierbare Substituenten, die in $\beta$-Stellung der Ethylgruppe von Y bzw. $Y^o$ stehen, sind beispielsweise Halogenatome, wie Brom und Chlor, Estergruppen organischer Carbon- und Sulfonsäuren, wie von Alkylcarbonsäuren, gegebenenfalls substituierter Benzolcarbonsäuren und gegebenenfalls substituierter Benzolsulfonsäuren, wie die Gruppen Alkanoyloxy von 2 bis 5 C-Atomen, hiervon insbesondere Acetyloxy, Benzoyloxy, Sulfobenzoyloxy, Phenylsulfonyloxy und Toluylsulfonyloxy, des weiteren saure Estergruppen anorganischer Säuren, wie der Phosphorsäure, Schwefelsäure und Thioschwefelsäure (Phosphato-, Sulfato- und Thiosulfatogruppen), ebenso Dialkylaminogruppen mit Alkylgruppen von jeweils 1 bis 4 C-Atomen, wie Dimethylamino und Diethylamino. Bevorzugt ist Y $\beta$-Sulfatoethyl oder Vinyl und insbesondere bevorzugt $\beta$-Chlorethyl, und bevorzugt ist $Y^o$ gleich $\beta$-Sulfatoethyl oder Vinyl, insbesondere bevorzugt $\beta$-Sulfatoethyl.

Der Farbstoffrest F, bzw. dessen Komponenten D, E, K und $K^o$ in den Formeln (1a) und (1b) insgesamt, besitzen bevorzugt eine oder mehrere, wie 2 bis 6, Sulfogruppen. Der Rest F kann weitere bei organischen Farbstoffen übliche Substituenten enthalten. Solche Substituenten sind beispielsweise: Alkylgruppen von 1 bis 4 C-Atomen, wie Methyl, Ethyl, Propyl, Isopropyl oder Butyl, hiervon bevorzugt Ethyl und insbesondere Methyl; Alkoxygruppen von 1 bis 4 C-Atomen, wie Methoxy, Ethoxy, Propoxy, Isopropoxy und Butoxy, bevorzugt hiervon Ethoxy und insbesondere Methoxy; Alkanoylaminogruppen von 2 bis 5 C-Atomen, wie die Acetylamino- und Propionylaminogruppe; gegebenenfalls durch Sulfo, Carboxy, Methyl, Ethyl, Methoxy, Ethoxy und/oder Chlor substituierte Benzoylaminogruppen; primäre und mono- oder disubstituierte Aminogruppen, wobei die Substituenten beispielsweise Alkylgruppen von 1 bis 4 C-Atomen und/oder Phenylgruppen sind, wie Monoalkylamino- und Dialkylaminogruppen mit 1 bis 4 C-Atomen im Alkylrest, Phenylamino- oder $N-(C_1-C_4\text{-Alkyl})$-N-phenyl-amino-Gruppen, wobei die Alkylreste noch substituiert sein können, beispielsweise durch Phenyl, Sulfophenyl, Hydroxy, Sulfato, Sulfo und Carboxy, und die Phenylgruppen noch substituiert sein können, wie durch Chlor, Sulfo, Carboxy, Methyl und/oder Methoxy, so beispielsweise Methylamino-, Ethylamino-, Propylamino-, Isopropylamino-, Butylamino-, N,N-Di-($\beta$-hydroxyethyl)-amino-, N,N-Di-($\beta$-sulfatoethyl)-amino-, Sulfobenzylamino-, N,N-Di-(sulfobenzyl)-amino- und Diethylaminogruppen sowie Phenylamino- und Sulfophenylaminogruppen; Alkoxycarbonylgruppen mit einem Alkylrest von 1 bis 4 C-Atomen, wie Methoxycarbonyl und Ethoxycarbonyl; Alkylsulfonylgruppen von 1 bis 4 C-Atomen, wie Methylsulfonyl und Ethylsulfonyl; Trifluormethyl-, Nitro- und Cyanogruppen; Halogenatome, wie Fluor, Chlor und Brom; Carbamoylgruppen, die durch Alkyl von 1 bis 4 C-Atomen mono- und disubstituiert sein können, wobei die Alkylreste wiederum substituiert sein können, beispielsweise durch Hydroxy, Sulfato, Sulfo, Carboxy, Phenyl und Sulfophenyl, wie beispielsweise N-Methyl-carbamoyl und N-Ethyl-carbamoyl; Sulfamoylgruppen, die durch Alkylgruppen von 1 bis 4 C-Atomen mono- oder disubstituiert sein können, und N-Phenyl-N-alkyl-sulfamoylgruppen mit einer Alkylgruppe von 1 bis 4 C-Atomen, wobei diese Alkylgruppen wiederum durch Hydroxy, Sulfato, Sulfo, Carboxy, Phenyl und Sulfophenyl substituiert sein können, wie beispielsweise N-Methyl-sulfamoyl, N-Ethyl-sulfamoyl, N-Propyl-sulfamoyl, N-Isopropyl-sulfamoyl, N-Butyl-sulfamoyl, N-(β-Hydroxyethyl)-sulfamoyl und N,N-Di-(β-hydroxyethyl)-sulfamoyl; N-Phenyl-sulfamoyl-,Ureido-, Hydroxy-, Carboxy-, Sulfomethyl- und Sulfogruppen.

In den allgemeinen Formeln (1) und (2) sowie in den nachfolgenden allgemeinen Formeln können die einzelnen Formelglieder, sowohl verschiedener als auch gleicher Bezeichnung innerhalb einer allgemeinen Formel, im Rahmen ihrer Bedeutung zueinander gleiche oder voneinander verschiedene Bedeutungen haben.

Die Gruppen "Sulfo", "Carboxy", "Phosphato" und "Sulfato" schließen sowohl deren Säureform als auch deren Salzform ein. Demgemäß bedeuten Sulfogruppen Gruppen entsprechend der allgemeinen Formel $-SO_3M$, Thiosulfatogruppen Gruppen entsprechend der allgemeinen Formel $-S-SO_3M$, Carboxygruppen Gruppen entsprechend der allgemeinen Formel -COOM, Phosphatogruppen Gruppen entsprechend der allgemeinen Formel $-OPO_3M_2$

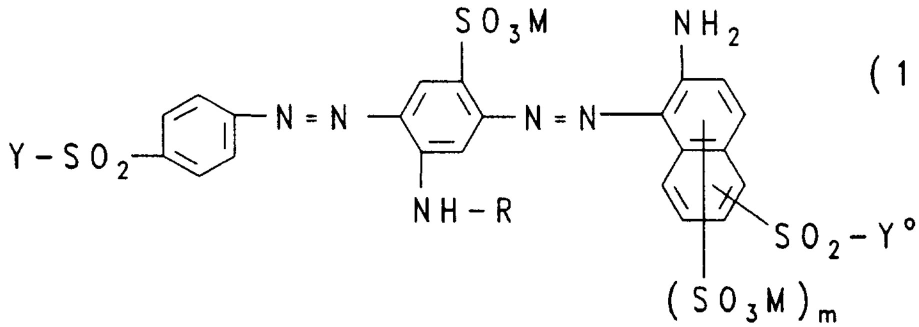

(1B)

In der Regel liegen die Disazoverbindungen der allgemeinen Formel (1A) und die Disazoverbindungen der allgemeinen Formel (1B) in einem Mischungsverhältnis von 5:95 bis 95:5 Gew.-%, bevorzugt im Mischungsverhältnis von 30:70 bis 70:30 Gew.-%, vor. Diese Gemische können in fester Form, beispielsweise als Pulver, vorliegen oder in Form einer wäßrigen, bevorzugt konzentrierten, Lösung als "Flüssigpräparation", die gegebenenfalls eine Puffersubstanz enthält, die einen pH-Wert zwischen 3 und 7 einzuhalten vermag, wie sie beispielsweise aus den U.S.-Patentschriften Nrs. 4 072 463 und 4 448 583 bekannt sind.

Die vorliegende Erfindung betrifft weiterhin ein Verfahren zur Herstellung der erfindungsgemäßen Disazoverbindungen der allgemeinen Formel (1), das dadurch gekennzeichnet ist, daß man eine Verbindung der allgemeinen Formel (5)

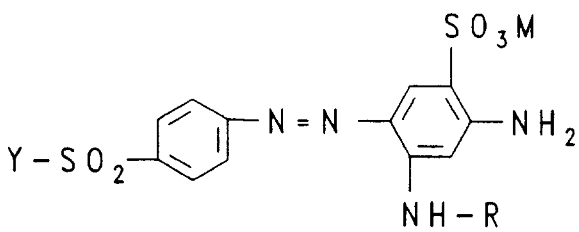

(5)

in welcher Y, R und M die obengenannten Bedeutungen haben, diazotiert und mit einer Verbindung der allgemeinen Formel (6) allgemeinen Formel (3a) oder (3b);

| | |
|---|---|
| Z-D²- | ist ein Rest der nachstehend angegebenen und definierten allgemeinen Formel (4a) oder (4b); |
| E | ist ein Rest der nachstehend angegebenen und definierten allgemeinen Formel (5a), (5b) oder (5c); |
| v | ist die Zahl Null oder 1; |
| $-K^1-R^G$ | ist eine Gruppe der nachstehend angegebenen und definierten allgemeinen Formel (6a), (6b), (6c), (6d), (6e), (6f), (6g) oder (6h); |
| $-K^2-Z$ | ist eine Gruppe der nachstehend angegebenen und definierten allgemeinen Formel (7a), (7b), (7c), (7d), (7e), (7f), (7g), (7h) oder (7i); |
| K° | ist der 1-Amino-8-hydroxy-3,6- oder -4,6-disulfo-naphth-2,7-ylen-Rest. |

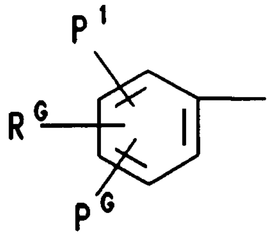

(3a)

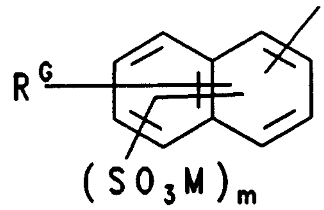

(3b)

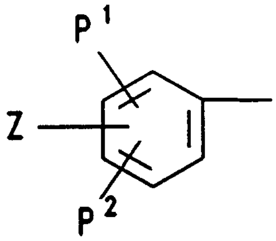

(4a)

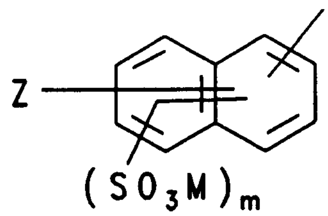

(4b)

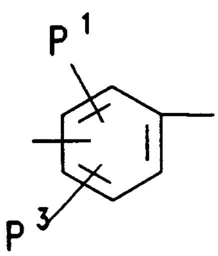

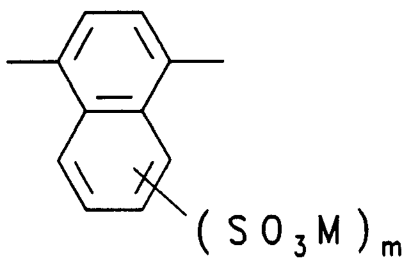

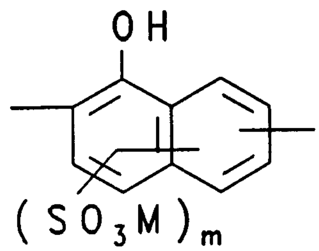

(5a) (5b) (5c)

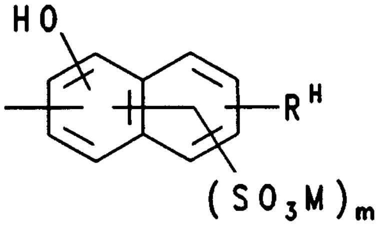

(6a)

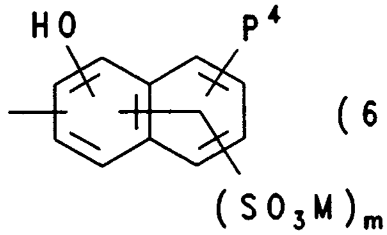

(6b)

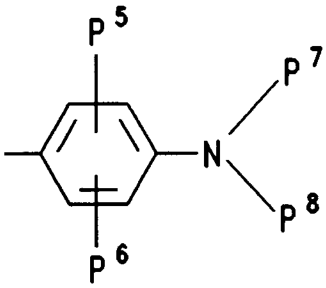

(6c)

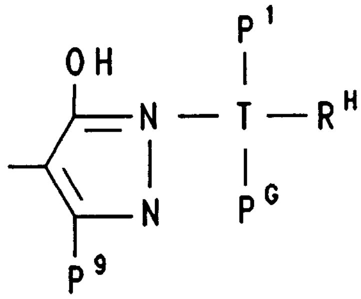

(6d)

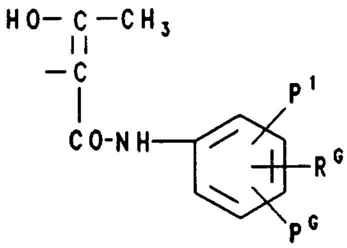

(6e)

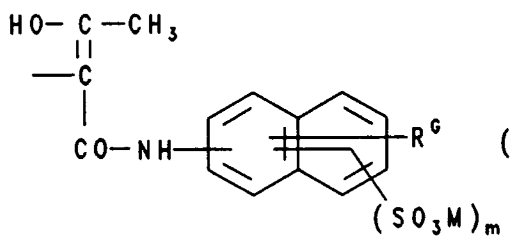

(6f)

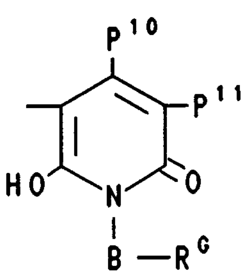

(6g)

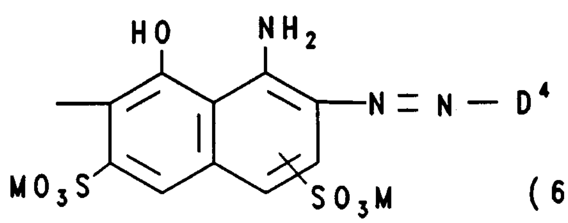

(6h)

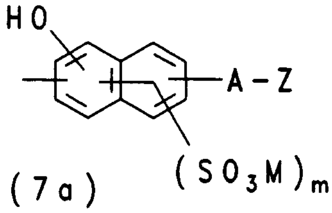

(7a)

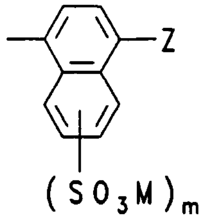

(7b)

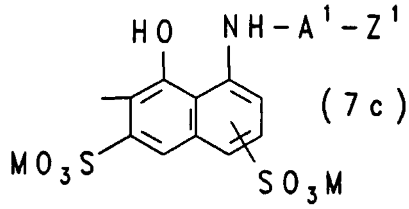

(7c)

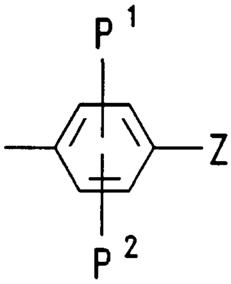

(7d)

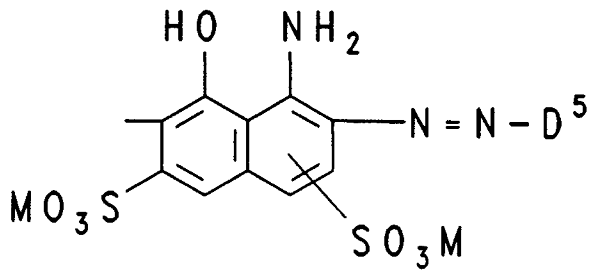

(7e)

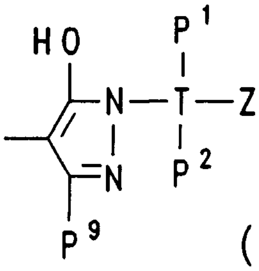

(7f)

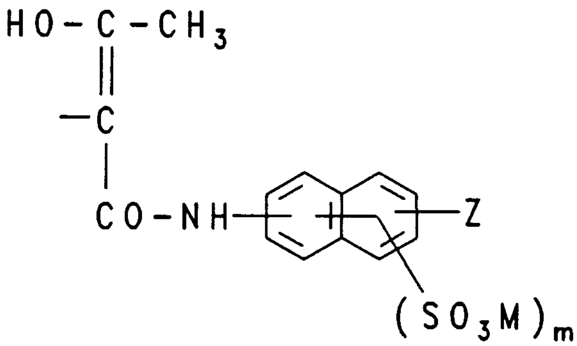

(7g)

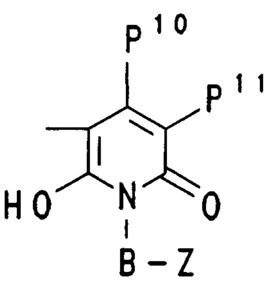

(7h)

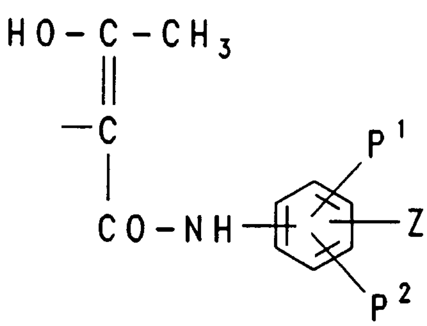

(7i)

In diesen Formeln bedeuten:

- M ist Wasserstoff oder ein Alkalimetall, wie Natrium, Kalium oder Lithium;
- Z hat eine der obengenannten, insbesondere bevorzugten Bedeutungen;
- $Z^1$ ist ein Rest der allgemeinen Formel (2A)

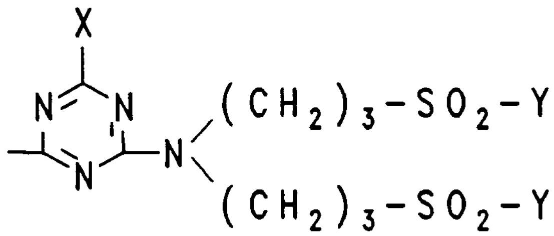

(2A)

in welcher X und Y die obengenannten, insbesondere bevorzugten, Bedeutungen haben;

$R^G$ ist eine Gruppe der allgemeinen Formel $Y^o$-$SO_2$-$W^o$-, in welcher $Y^o$ und $W^o$ eine der obengenannten Bedeutungen haben, oder ist eine Gruppe der allgemeinen Formel $R^G$-$D^3$-N = N-, in welcher $R^G$ hier einen Rest der oben definierten allgemeinen Formel $Y^o$-$SO_2$-$W^o$- bedeutet und $D^3$ einen Phenylenrest darstellt, der die später definierten Substituenten $P^1$ und $P^G$ enthält, oder einen Naphthylenrest bedeutet, der durch 1 oder 2 Sulfogruppen substituiert ist;

$P^1$ ist Wasserstoff, Methyl, Ethyl, Methoxy, Ethoxy, Hydroxy, Alkanoyl von 2 bis 5 C-Atomen, wie Acetyl und Propionyl, Cyano, Sulfo, Carboxy, Nitro, Phenylamino, Phenoxy, Sulfophenylamino, Alkoxycarbonyl von 2 bis 5 C-Atomen, wie Methoxycarbonyl und Ethoxycarbonyl, Sulfamoyl, Carbamoyl, N-($C_1$-$C_4$-Alkyl)-carbamoyl, Fluor, Chlor, Brom oder Trifluormethyl;

$P^2$ ist Wasserstoff, Methyl, Ethyl, Methoxy, Ethoxy, Cyano, Carboxy, Sulfo, Chlor, Alkanoylamino von 2 bis 5 C-Atomen, wie Acetylamino, Alkoxycarbonyl von 2 bis 5 C-Atomen, wie Methoxycarbonyl und Ethoxycarbonyl, Carbamoyl, N-($C_1$-$C_4$-Alkyl)-sulfamoyl, N-Sulfophenyl-amidocarbonyl, N-Phenyl-amidocarbonyl, Alkylsulfonyl von 1 bis 4 C-Atomen, Phenylsulfonyl oder Phenoxy;

$P^G$ ist Wasserstoff, Methyl, Ethyl, Methoxy, Ethoxy, Cyano, Carboxy, Sulfo, Chlor, Alkanoylamino von 2 bis 5 C-Atomen, wie Acetylamino, Alkoxycarbonyl von 2 bis 5 C-Atomen, wie Methoxycarbonyl und Ethoxycarbonyl, Carbamoyl, N-($C_1$-$C_4$-Alkyl)-sulfamoyl, N-Sulfophenyl-amidocarbonyl, N-Phenyl-amidocarbonyl, Alkylsulfonyl von 1 bis 4 C-Atomen, Phenylsulfonyl oder Phenoxy oder ist eine Gruppe der oben definierten allgemeinen Formel $Y^o$-$SO_2$-$W^o$-;

m steht für die Zahl Null, 1 oder 2 (wobei diese Gruppe im Falle von m gleich Null ein Wasserstoffatom bedeutet);

$P^3$ ist Wasserstoff, Alkyl von 1 bis 4 C-Atomen, wie Methyl oder Ethyl, Alkoxy von 1 bis 4 C-Atomen, wie Methoxy und Ethoxy, Chlor, Amino, Alkanoylamino von 2 bis 5 C-Atomen, wie Acetylamino und Propionylamino, Benzoylamino, Ureido, Phenylureido, Alkylureido mit 1 bis 4 C-Atomen im Alkylrest, Phenylsulfonyl oder Alkylsulfonyl von 1 bis 4 C-Atomen;

$R^H$ ist eine Gruppe der oben definierten allgemeinen Formel -$W^o$-$SO_2$-$Y^o$ oder eine Gruppe der allgemeinen Formel -N = N-$K^3$, in welcher $K^3$ eine Gruppe der oben angegebenen allgemeinen Formel (6c), (6d), (6e) oder (6h) ist, in welchen $R^H$ jedoch hier eine Gruppe der oben definierten allgemeinen Formel -$W^o$-$SO_2$-$Y^o$ ist;

$P^4$ ist Phenylureido, das im Phenylrest durch Substituenten aus der Gruppe Chlor, Methyl, Methoxy, Sulfo und Carboxy substituiert sein kann und durch eine Gruppe der oben definierten allgemeinen Formel -$W^o$-$SO_2$-$Y^o$ substituiert ist, oder ist Alkanoylamino von 2 bis 5 C-Atomen, wie Acetylamino oder Propionylamino, oder ist Benzoylamino, das im Benzolrest durch Substituenten aus der Gruppe Chlor, Methyl, Methoxy, Nitro, Sulfo und Carboxy substituiert sein kann und durch eine Gruppe der oben definierten allgemeinen Formel -$W^o$-$SO_2$-$Y^o$ substituiert ist;

$P^5$ ist Wasserstoff, Alkyl von 1 bis 4 C-Atomen, wie Methyl und Ethyl, Alkoxy von 1 bis 4 C-Atomen, wie Methoxy und Ethoxy, Brom, Chlor oder Alkanoylamino von 2 bis 7 C-Atomen, wie Acetylamino und Propionylamino;

$P^6$ ist Alkyl von 1 bis 4 C-Atomen, wie Methyl und Ethyl, Alkoxy von 1 bis 4 C-Atomen, wie Methoxy und Ethoxy, Chlor oder Alkanoylamino von 2 bis 7 C-Atomen, wie Acetylamino und Propionylamino, Ureido oder Phenylureido;

$P^7$ ist Wasserstofff oder Alkyl von 1 bis 4 C-Atomen, das durch Hydroxy, Cyano, Carboxy, Sulfo, Sulfato, Methoxycarbonyl, Ethoxycarbonyl oder Acetoxy oder eine Gruppe der oben definierten allgemeinen Formel -$SO_2$-$Y^o$ substituiert sein kann;

$P^8$ ist Alkyl von 1 bis 4 C-Atomen, das durch Hydroxy, Cyano, Carboxy, Sulfo, Sulfato, Methoxycarbonyl, Ethoxycarbonyl oder Acetoxy oder eine Gruppe der oben definierten allgemeinen Formel -$SO_2$-$Y^o$ substituiert sein kann, oder ist Benzyl oder Phenyl oder durch Alkyl von 1 bis 4 C-Atomen, Alkoxy von 1 bis 4 C-Atomen, Chlor und/oder Sulfo substituiertes Benzyl oder Phenyl,

wobei diese Phenylreste auch durch eine Gruppe der oben definierten allgemeinen Formel $-W^o-SO_2-Y^o$ substituiert sein können und wobei mindestens einer der Reste $P^7$ und $P^8$ einen Rest der allgemeinen Formel $-SO_2-Y^o$ enthält;

$P^9$ ist Wasserstoff, Alkyl von 1 bis 4 C-Atomen, wie Methyl, Cyano, Carboxy, Carbalkoxy von 2 bis 5 C-Atomen, wie Carbomethoxy und Carbethoxy, Carbamoyl oder Phenyl, bevorzugt Methyl, Carboxy, Methoxycarbonyl, Ethoxycarbonyl oder Phenyl und insbesondere Methyl oder Carboxy;

T steht für einen Benzol- oder Naphthalinring, bevorzugt einen Benzolring;

$P^{10}$ ist Wasserstoff oder Alkyl von 1 bis 4 C-Atomen, wie Methyl, oder durch Alkoxy von 1 bis 4 C-Atomen, wie Methoxy, oder Cyano substituiertes Alkyl von 1 bis 4 C-Atomen oder ist Phenyl, bevorzugt Alkyl von 1 bis 4 C-Atomen oder Phenyl;

$P^{11}$ ist Wasserstoff, Chlor, Brom, Sulfo, Carbamoyl, Methylsulfonyl, Phenylsulfonyl, Cyano oder Sulfoalkyl von 1 bis 4 C-Atomen, bevorzugt Wasserstoff, Sulfo, Sulfoalkyl mit einem Alkylrest von 1 bis 4 C-Atomen, wie Sulfomethyl, Cyano oder Carbamoyl;

A ist ein Rest der Formel -NH-phenylen- oder -NH-CO-phenylen- oder -NH-CO-NH-phenylen- oder eine direkte, kovalente Bindung;

$A^1$ ist ein Rest der Formel -phenylen-NH- oder -CO-phenylen-NH- oder -CO-NH-phenylen-NH- oder eine kovalente Bindung;

B ist Alkylen von 1 bis 4 C-Atomen, das durch Hydroxy, Sulfato, Sulfo, Carboxy, Phosphato oder Acetyloxy substituiert sein kann, oder ist Methylenphenylen, Ethylenphenylen, Phenylenmethylen, Phenylenethylen oder Phenylen oder im Benzolrest durch Fluor, Chlor, Brom, Methyl, Methoxy, Cyano, Sulfo, Carboxy, Acetyl, Nitro, Carbamoyl und/oder Sulfamoyl substituiertes Methylenphenylen, Ethylenphenylen oder Phenylen;

$D^4$ ist ein Rest der oben angegebenen allgemeinen Formel (3a) oder (3b), in welchen $P^1$ und $P^2$ eine der obengenannten Bedeutungen besitzt und $R^G$ hier eine Gruppe der oben definierten allgemeinen Formel $-W^o-SO_2-Y^o$ ist;

$D^5$ ist eine Gruppe der oben genannten und definierten allgemeinen Formel (4a) oder (4b);

in den Formeln (6a), (6b) und (7a) steht die freie Bindung, die zur Azogruppe führt, in ortho-Stellung zur Hydroxygruppe an den aromatischen Kern gebunden, und in Formel (5c) steht die freie Bindung, die zm Rest $-N = N-K^1-R^G$ oder $-N = N-K^2-Z$ führt, in 2- oder 3-Stellung an den 8-Hydroxy-naphthyl-Rest gebunden, und in den Formeln (6a) und (6f) steht $R^H$, falls $R^H$ eine Gruppe $-N = N-K^3$ bedeutet, sowie in Formel (7a) der Rest Z in $\beta$-Stellung des jeweiligen Naphthalinrestes gebunden.

Bevorzugt ist hiervon $P^1$ gleich Wasserstoff, Methyl, Methoxy, Brom, Chlor, Sulfo und Carboxy sowie $P^2$ gleich Wasserstoff, Methyl, Methoxy, Chlor, Carboxy, Sulfo und Acetylamino.

Von den Resten der Formeln (7) sind bevorzugt Reste entsprechend den allgemeinen Formeln (8a), (8b), (8c), (8d), (8e), (8f), (8g) und (8h)

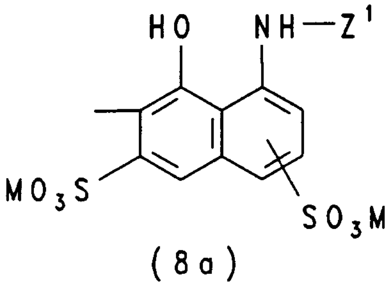

(8a)

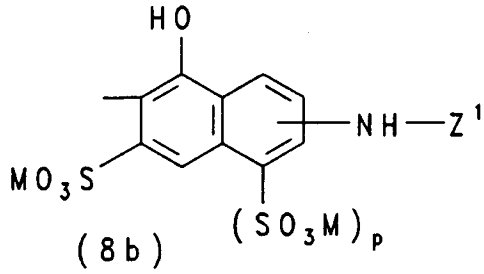

(8b)

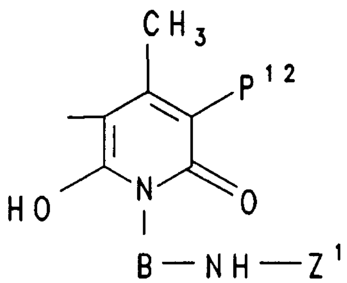

(8c)

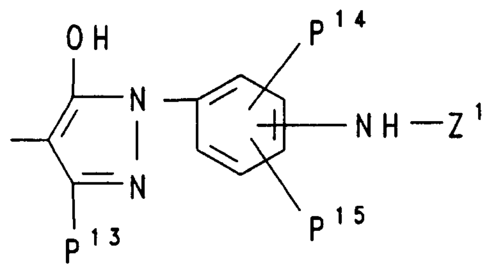

(8d)

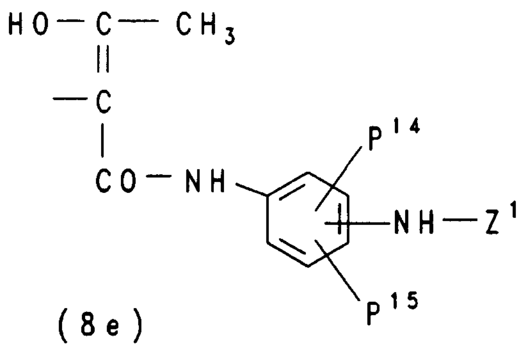

(8e)

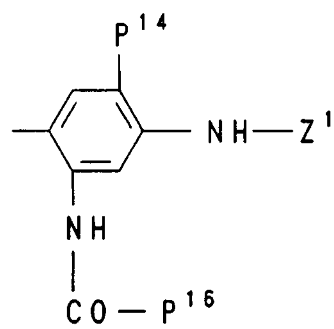

(8f)

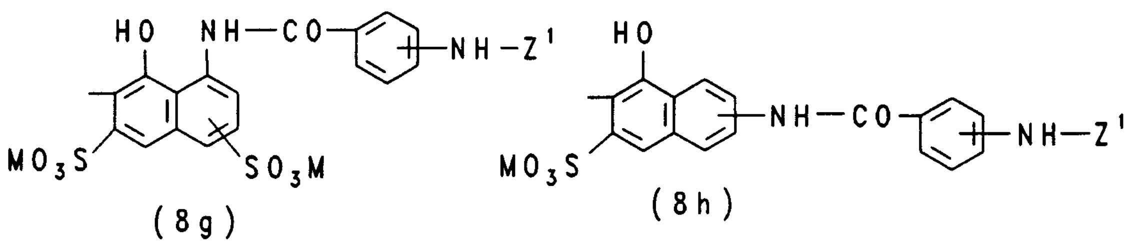

(8g)

(8h)

in welchen

M, $P^{11}$ und $Z^1$ eine der obengenannten Bedeutungen, insbesondere bevorzugten Bedeutungen, besitzen,

p die Zahl Null oder 1 ist (wobei im Falle p gleich Null diese Gruppe Wasserstoff bedeutet),

| | |
|---|---|
| B | Alkylen von 2 bis 4 C-Atomen oder Phenylen ist, |
| $P^{12}$ | Wasserstoff, Sulfo, Sulfoalkyl von 1 bis 4 C-Atomen, wie Sulfomethyl, Cyano oder Carbamoyl ist, |
| $P^{13}$ | Carboxy, Methyl oder Carbethoxy ist, |
| $P^{14}$ | Wasserstoff, Methyl, Methoxy, Sulfo oder Chlor ist, |
| $P^{15}$ | Wasserstoff, Methyl oder Methoxy ist, |
| $P^{16}$ | Methyl oder Amino ist und die Aminogruppe in den Formeln (8b) und (8h) sich in 2- oder 3-Stellung des 8-Naphtholrestes befindet. |

Bevorzugt hiervon sind insbesondere Reste der allgemeinen Formeln (8a), (8b), (8d) und (8f).

Von den Azofarbstoffen der allgemeinen Formel (1e) sind insbesondere die Disazofarbstoffe der allgemeinen Formeln (1f) und (1g)

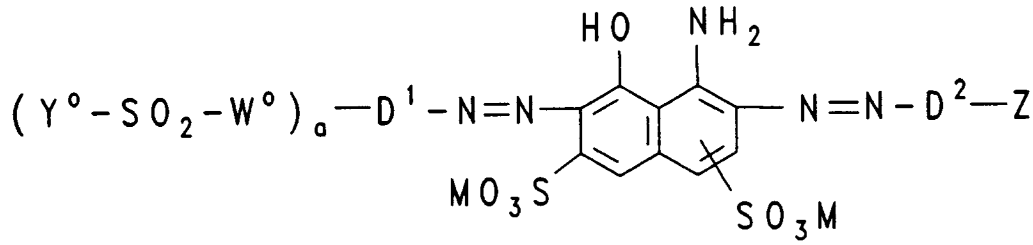

(1f)

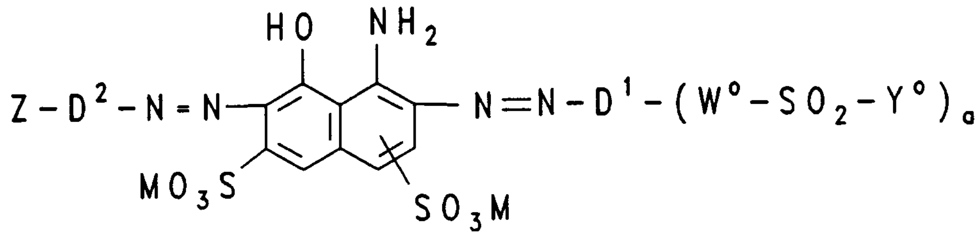

(1g)

hervorzuheben, in welchen M, Y°, W°, a, $D^1$, $D^2$ und Z die obengenannten Bedeutungen haben.

Gruppen der allgemeinen Formel $R^G$-D- bzw. der allgemeinen Formeln (3a) und (3b) sind beispielsweise:

2-(β-Sulfatoethylsulfonyl)-phenyl, 3-(β-Sulfatoethylsulfonyl)-phenyl,
4-(β-Sulfatoethylsulfonyl)-phenyl, 2-Carboxy-5-(β-sulfatoethylsulfonyl)-phenyl,
2-Chlor-3-(sulfatoethylsulfonyl)-phenyl, 2-Chlor-4-(β-sulfatoethylsulfonyl)-phenyl, 2-Brom-4-(β-sulfatoethylsulfonyl)-phenyl, 4-Methoxy-3-(β-sulfatoethylsulfonyl)-phenyl, 4-Chlor-3-(β-sulfatoethylsulfonyl)-phenyl, 2-Ethoxy-4- oder -5-(β-sulfatoethylsulfonyl)-phenyl, 2-Methyl-4-(β-sulfatoethylsulfonyl)-phenyl, 2-Methoxy-5- oder -4-(β-sulfatoethylsulfonyl)-phenyl, 2,4-Diethoxy-5-(β-sulfatoethylsulfonyl)-phenyl, 2,4-Dimethoxy-5-(β-sulfatoethylsulfonyl)-phenyl,
2,5-Dimethoxy-4-(β-sulfatoethylsulfonyl)-phenyl, 2-Methoxy-5-methyl-4-(β-sulfatoethylsulfonyl)-phenyl, 2- oder 3- oder 4-(β-Thiosulfatoethylsulfonyl)-phenyl, 2-Methoxy-5-(β-thiosulfatoethylsulfonyl)-phenyl, 2-Sulfo-4-(β-phosphatoethylsulfonyl)-phenyl, 2-Sulfo-4-vinylsulfonyl-phenyl, 2-Hydroxy-4- oder -5-(β-sulfatoethylsulfonyl)-phenyl, 2-Chlor-4- oder -5-(β-chlorethylsulfonyl)-phenyl, 2-Hydroxy-5-(β-sulfatoethylsulfonyl)-phenyl,
2-Hydroxy-3-sulfo-5-(β-sulfatoethylsulfonyl)-phenyl, 3- oder 4-(β-Acetoxyethylsulfonyl)-phenyl,
6-Carboxy-1-sulfo-naphth-2-yl, 5-(β-Sulfatoethylsulfonyl)-naphth-2-yl, 6- oder 7- oder 8-(β-Sulfatoethylsulfonyl]-naphth-2-yl, 6-(β-Sulfatoethylsulfonyl)-1-sulfo-naphth-2-yl, 5-(β-Sulfatoethylsulfonyl)-1-sulfo-naphth-2-yl, 8-(β-Sulfatoethylsulfonyl)-6-sulfo-naphth-2-yl, 4-[β-(β'-Sulfatoethylsulfonyl)-ethyl]-phenyl, 3- oder 4-[β-(β'-Chlorethylsulfonyl)-ethylamino]-phenyl, 3- oder
4-[β-(β'-Sulfatoethylsulfonyl)-ethylamino]-phenyl, 3- oder 4-[γ-(β'-Chlorethylsulfonyl)-propylamino]-phenyl, 3- oder 4-[γ-(β'-Sulfatoethylsulfonyl)-propylamino]-phenyl, 3- oder 4-[γ-(Vinylsulfonyl)-propylamino]-phenyl,
4-[β-(β'-Sulfatoethylsulfonyl)-ethylamino]-2- oder -3-sulfo-phenyl,
4-[β-(β'-Chlorethylsulfonyl)-ethylamino]-2- oder -3-sulfo-phenyl,
4-[γ-(β'-Sulfatoethylsulfonyl)-propylamino]-2- oder -3-sulfo-phenyl,
4-[γ-(β'-Chlorethylsulfonyl)-propylamino]-2- oder -3-sulfo-phenyl,

4-[β-(β'-Chlorethylsulfonyl)-ethylamino]-2-carboxy-phenyl,
4-[β-(β'-Sulfatoethylsulfonyl)-ethylamino]-2-carboxy-phenyl,
4-[γ-(β'-Chlorethylsulfonyl)-propylamino]-2-carboxy-phenyl und
4-[γ-(β'-Sulfatoethylsulfonyl)-propylamino]-2-carboxy-phenyl.

Gruppen entsprechend den allgemeinen Formelresten $R^G$-D-N = N-E- bzw. $R^G$-$D^1$-N = N-E- sind beispielsweise 4-[4'-(β-Sulfatoethylsulfonyl)-phenyl]-azo-2-methyl-5-methoxy-phenyl, 4-[3'-(β'-Sulfatoethylsulfonyl)-phenyl]-azo-3-methyl-phenyl, 4-[4'-(β-Sulfatoethylsulfonyl)-phenyl]-azo-3-ureido-phenyl, 4-[6'-(β-Sulfatoethylsulfonyl)-naphth-2'-yl]-azo-3-ureido-phenyl und 7-[2'-Methoxy-5'-(β-sulfatoethylsulfonyl)-phenyl]-azo-8-hydroxy-6-sulfo-naphth-3-yl.

Von den Azofarbstoffen sind weiterhin bevorzugt solche, die den allgemeinen Formeln (12A) bis (12S)

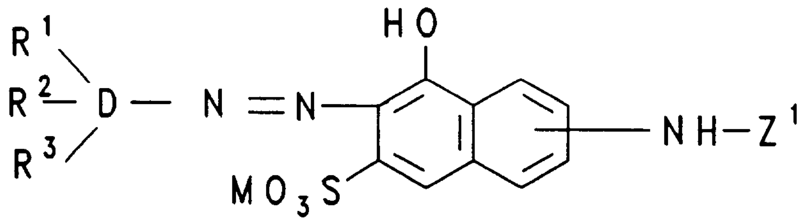

(12A)

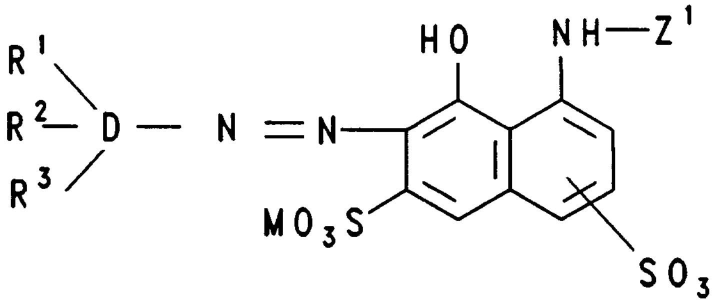

(12B)

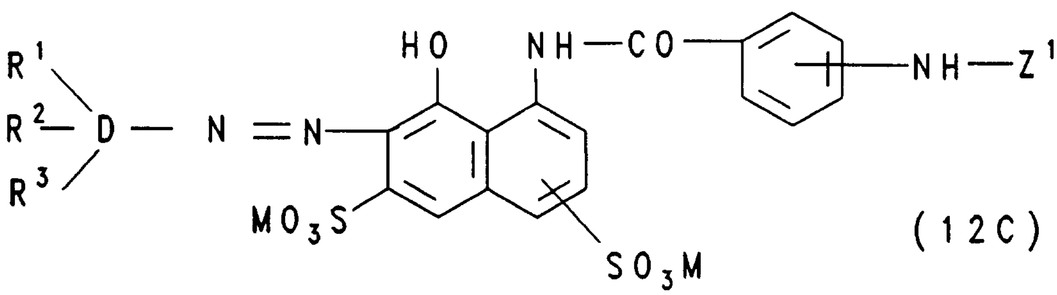

(12C)

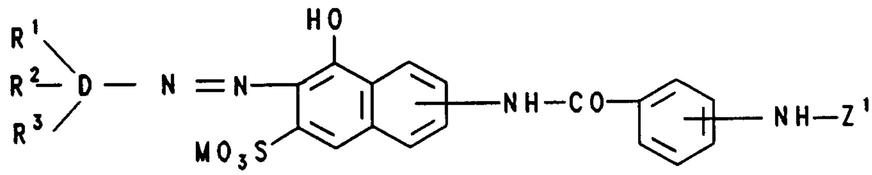

(12D)

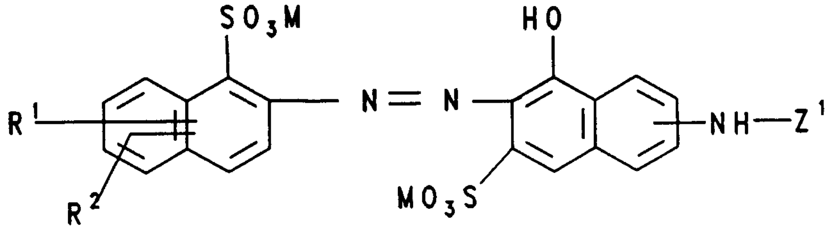

(12E)

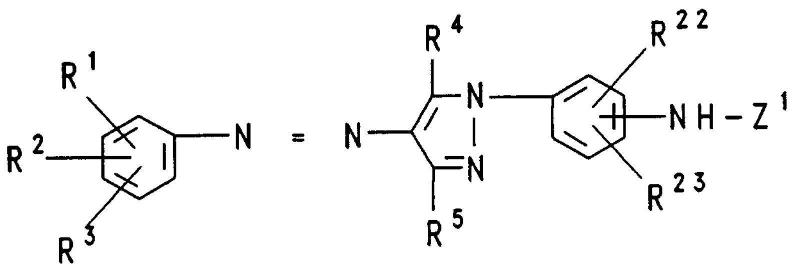

(12F)

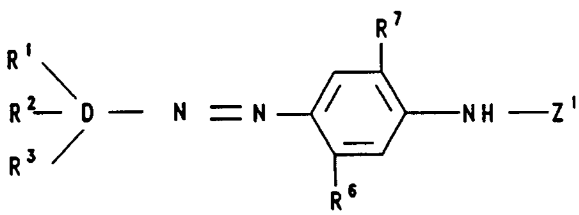

(12G)

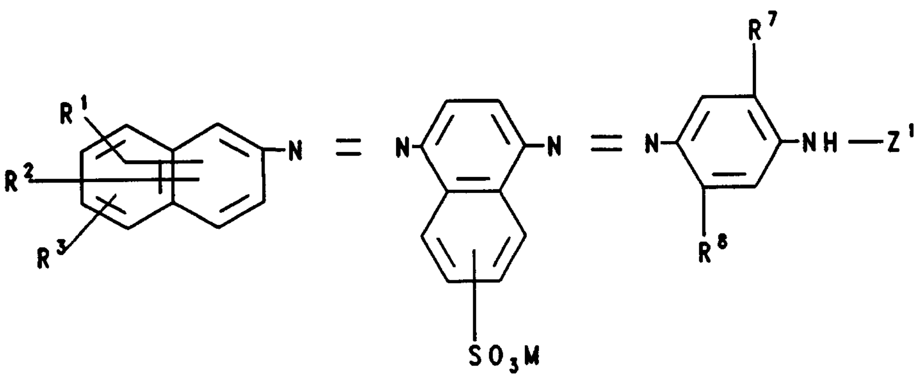

(12H)

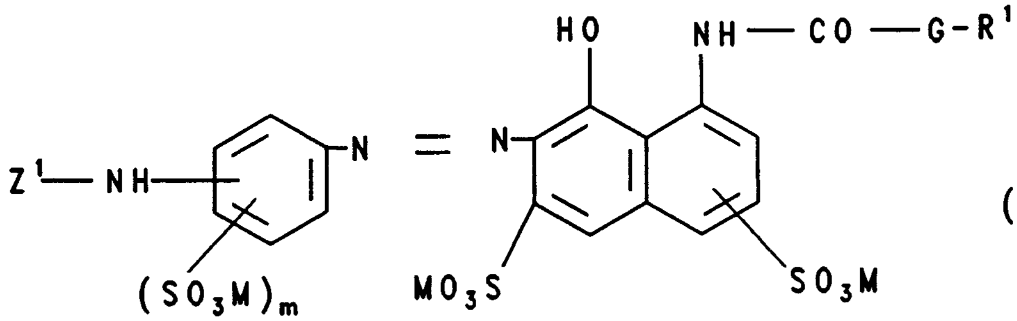

(12J)

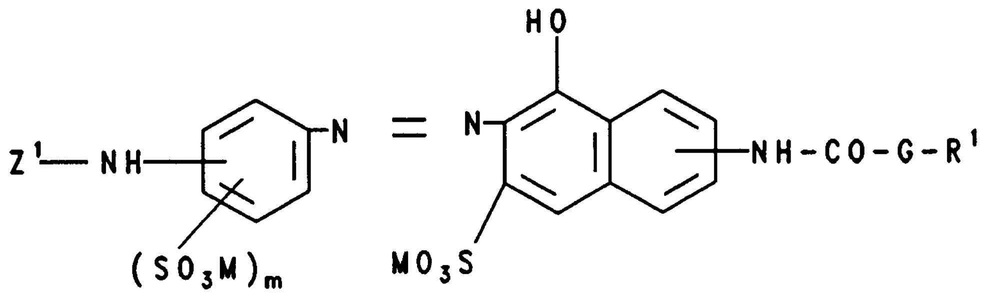

(12K)

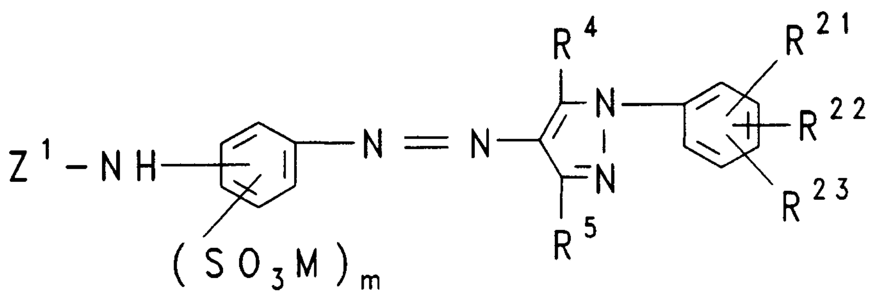

(12L)

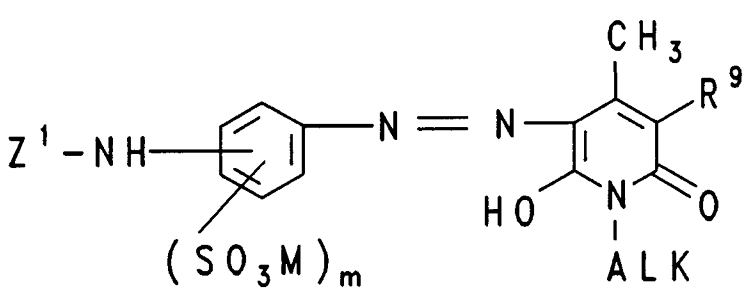

(12M)

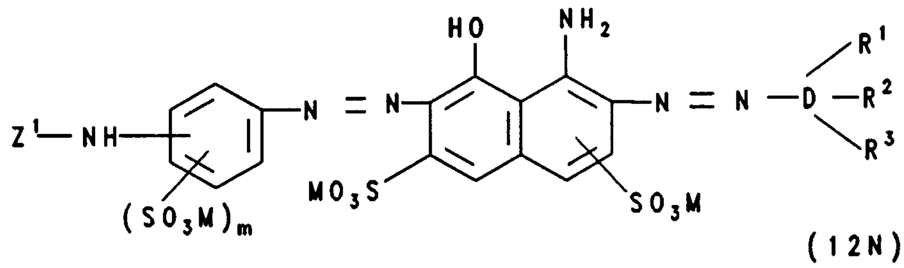

(12N)

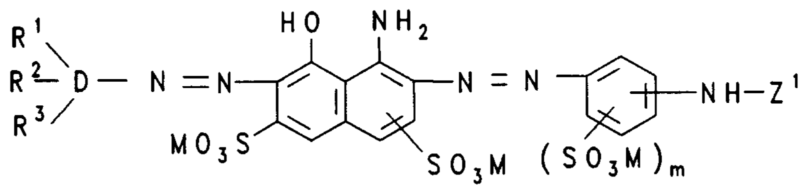

(12P)

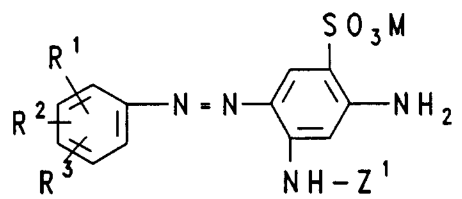

(12Q)

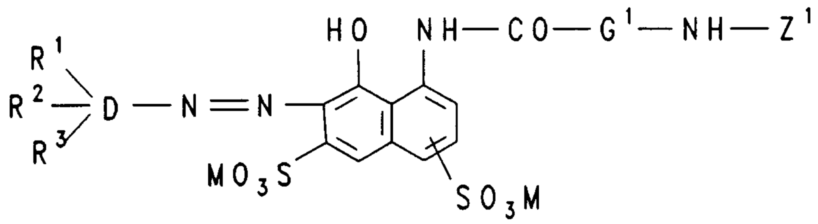

(12R)

(12S)

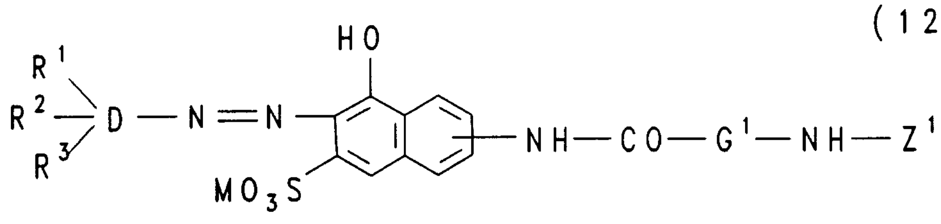

entsprechen, in welchen bedeuten:

M und $Z^1$ haben eine der obengenannten Bedeutungen;

D ist ein Benzolring oder ist ein Naphthalinring, wobei die Azogruppe an den Naphthalinring bevorzugt in $\beta$-Stellung gebunden ist und wobei im Falle, daß D den Naphthalinring bedeutet, $R^2$ und $R^3$ bevorzugt jedes, unabhängig voneinander, ein Wasserstoffatom oder

eine Sulfogruppe bedeuten;

$R^1$ ist eine Gruppe $Y^o$-$SO_2$-$W^o$- obengenannter Bedeutung, wobei $W^o$ bevorzugt eine kovalente Bindung ist;

$R^2$ ist Wasserstoff, Alkyl von 1 bis 4 C-Atomen, wie Ethyl und insbesondere Methyl, Alkoxy von 1 bis 4 C-Atomen, wie Ethoxy und insbesondere Methoxy, Halogen, wie Chlor und Brom, Carboxy oder Sulfo und ist bevorzugt Wasserstoff, Methyl, Methoxy, Brom, Chlor, Sulfo oder Carboxy und insbesondere bevorzugt Wasserstoff, Methoxy oder Sulfo;

$R^3$ ist Wasserstoff, Alkyl von 1 bis 4 C-Atomen, wie Ethyl und insbesondere Methyl, Alkoxy von 1 bis 4 C-Atomen, wie Ethoxy und insbesondere Methoxy, Halogen, wie Chlor und Brom, Nitro, Carboxy oder Sulfo, bevorzugt Wasserstoff, Methyl, Methoxy, Chlor, Carboxy, Sulfo oder Acetylamino und insbesondere bevorzugt Wasserstoff, Methoxy oder Sulfo;

$R^4$ ist Hydroxy oder Amino, bevorzugt Hydroxy;

$R^5$ ist Methyl, Carboxy, Carbomethoxy oder Carbethoxy, bevorzugt Methyl oder Carboxy;

$R^6$ ist Acetylamino, Propionylamino, Ureido oder Methyl;

$R^7$ ist Wasserstoff, Alkyl von 1 bis 4 C-Atomen, wie Methyl und Ethyl, Alkoxy von 1 bis 4 C-Atomen, wie Methoxy und Ethoxy, Halogen, wie Brom und insbesondere Chlor, vorzugsweise Wasserstoff, Methyl, Ethyl, Methoxy oder Ethoxy;

$R^8$ ist Wasserstoff oder Alkyl von 1 bis 4 C-Atomen, wie Methyl und Ethyl, Alkanoylamino von 2 bis 5 C-Atomen, wie Acetylamino und Propionylamino, oder Ureido, vorzugsweise Wasserstoff, Methyl, Ethyl, Methoxy, Ethoxy, Acetylamino oder Ureido;

$R^9$ ist Wasserstoff, Cyano, Carbamoyl, Sulfamoyl oder Sulfomethyl, bevorzugt Wasserstoff oder Carbamoyl;

$R^{21}$ hat eine der Bedeutungen von $R^1$;

$R^{22}$ hat eine der Bedeutungen von $R^2$;

$R^{23}$ hat eine der Bedeutungen von $R^3$;

ALK ist Alkyl von 1 bis 4 C-Atomen, wie Methyl und Ethyl, oder Alkyl von 2 bis 4 C-Atomen, wie Ethyl und Propyl, das durch Hydroxy, Carboxy, Sulfo oder Sulfato substituiert sein kann;

G ist Alkylen von 1 bis 4 C-Atomen, wie Ethylen und Propylen, oder ist Phenylen oder durch Sulfo, Carboxy und/oder Alkyl von 1 bis 4 C-Atomen, wie Methyl, substituiertes Phenylen;

$G^1$ ist eine Gruppe der Formel -NH-alk-, -NH-phen- oder -alk-, in welchen alk für Alkylen von 2 bis 4 C-Atomen steht und phen Sulfo-phenylen oder Phenylen bedeutet;

m ist die Zahl Null, 1 oder 2 (wobei im Falle von m gleich Null diese Gruppe Wasserstoff ist);

in den Verbindungen der Formeln (12A), (12D), (12E), (12K) und (12S) steht die Amino- bzw. Amidogruppierung in 2- oder 3-Stellung an den 8-Naphthol-Rest gebunden und in den Verbindungen der Formeln (12B), (12C), (12J), und (12R) steht die Gruppe $-SO_3M$ in meta- oder para-Stellung zur Amino- bzw. Acylaminogruppe gebunden.

Die vorliegende Erfindung betrifft weiterhin Verfahren zur Herstellung der erfindungsgemäßen Azofarbstoffe der allgemeinen Formel (1). Sie lassen sich in an und für sich in üblicher Weise analog bekannten Synthesewegen herstellen, indem man für den jeweiligen Farbstoff typische Vorprodukte, von denen mindestens eines eine Gruppe der Formel $Y^o$-$SO_2$- und mindestens eines eine Gruppe der allgemeinen Formel (2) enthält, miteinander umsetzt, oder indem man eine Halogen-triazin-Verbindung der allgemeinen Formel (21)

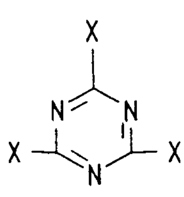

(21)

in welcher X eine der obengenannten Bedeutungen besitzt, wie beispielsweise 2,4,6-Trifluor-1,3,5-triazin (Cyanurfluorid) oder 2,4,6-Trichlor-1,3,5-triazin (Cyanurchlorid), mit einer aminogruppenhaltigen Ausgangsverbindung der allgemeinen Formel (20a) oder (20b)

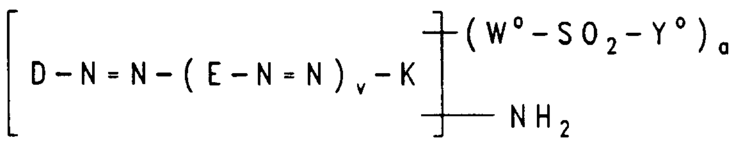

(20a)

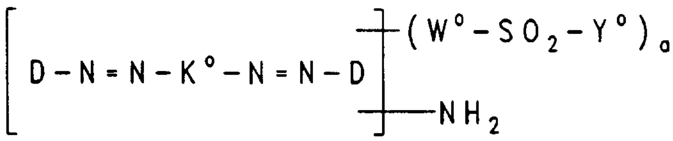

(20b)

in welchen D, E, K, $K^o$, v, $W^o$, $Y^o$ und a die obengenannten Bedeutungen haben, wobei in Formel (20a) die Gruppe(n) $-W^o-SO_2-Y^o$ an D und die Aminogruppe $-NH_2$ an K oder die Aminogruppe an D und die Gruppe(n) $-W^o-SO_2-Y^o$ an K gebunden und in Formel (20b) die Gruppe(n) $-W^o-SO_2-Y^o$ an das eine D und die Aminogruppe an das andere D gebunden sind,
und mit einer Aminoverbindung der allgemeinen Formel (22)

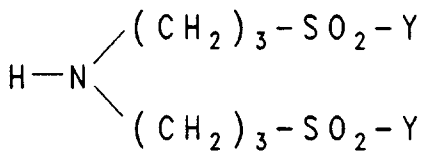

(22)

in welcher Y eine der obengenannten Bedeutungen hat, in beliebiger Reihenfolge miteinander umsetzt.

Erfindungsgemäße Verfahrensvarianten dieser erfindungsgemäßen Verfahrensweise sind beispielsweise dadurch gekennzeichnet, daß man eine Verbindung der allgemeinen Formel (20c) oder (20d)

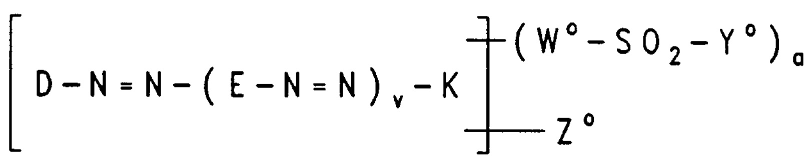

(20c)

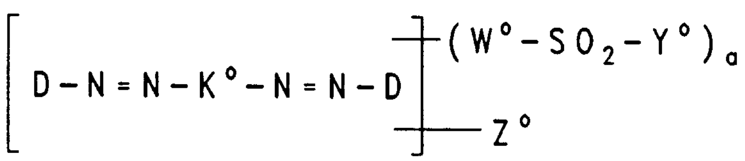

(20d)

in welchen D, E, K, $K^o$, v, $W^o$, $Y^o$ und a die obengenannten Bedeutungen haben und $Z^o$ ein Rest der allgemeinen Formel (23)

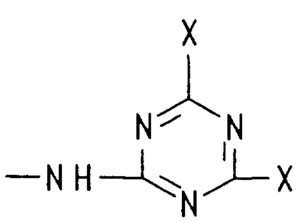

(23)

mit X der obengenannten Bedeutung ist, wobei in Formel (22c) die Gruppe(n) $-W^o-SO_2-Y^o$ an D und die Gruppe $Z^o$ an K oder die Gruppe $Z^o$ an D und die Gruppe(n) $-W^o-SO_2-Y^o$ an K und in Formel (22d) die Gruppe(n) $-W^o-SO_2-Y^o$ an das eine D und die Gruppe $Z^o$ an das andere D gebunden sind,
mit einer Aminoverbindung der allgemeinen Formel (22) umsetzt,
oder daß man eine Verbindung der allgemeinen Formel (24)

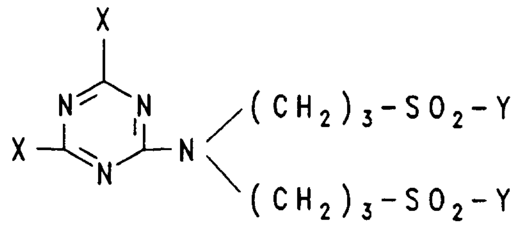

(24)

in welcher X und Y die obengenannten Bedeutungen haben, mit einer Verbindung der allgemeinen Formeln (20) der obengenannten Bedeutung umsetzt. Hiervon ist die Verfahrensweise der Umsetzung einer Verbindung der allgemeinen Formeln (23) mit einer Verbindung der allgemeinen Formel (22) bevorzugt.

Die Umsetzungen der Ausgangsverbindungen erfolgen im wäßrigen oder wäßrigorganischen Medium in Suspension oder Lösung. Führt man die Umsetzungen in einem wäßrig-organischen Medium durch, so ist das organische Medium beispielsweise Aceton, Dimethylformamid, Dimethylsulfoxid oder N-Methylpyrrolidon. Vorteilhaft wird der bei der Kondensation freiwerdende Halogenwasserstoff laufend durch Zugabe wäßriger Alkalihydroxide, -carbonate oder -bicarbonate neutralisiert. Die erste Kondensationsreaktion des Halogen-s-triazins der allgemeinen Formel (21) erfolgt in der Regel bei einer Temperatur zwischen 0 ° C und 60 ° C vorzugsweise zwischen 5 und 60 ° C, und bei einem pH-Wert zwischen 2 und 7, bevorzugt zwischen 3 und 6, im Falle von X gleich Chlor bevorzugt bei einer Temperatur zwischen 20 und 60 ° C und bei einem pH-Wert zwischen 3 und 6 und im Falle von X gleich Fluor bevorzugt bei einer Temperatur zwischen 5 ° C und 20 ° C und bei einem pH-Wert zwischen 4 und 5. Die nachfolgende Kondensationsreaktion mit der zweiten Aminoverbindung erfolgt in der Regel bei einer Temperatur zwischen 5 und 40 ° C, im Falle von X gleich Chlor bevorzugt bei 20 bis 40 ° C und im Falle von X gleich Fluor bevorzugt bei einer Temperatur zwischen 0 und 15 ° C, und bei einem pH-Wert zwischen 4 und 10.

Insbesondere erfolgt die Umsetzung zwischen einer Verbindung der allgemeinen Formeln (20c) oder (20d) und einer Verbindung der allgemeinen Formel (22) bei einer Temperatur zwischen 20 und 45 ° C, bevorzugt zwischen 30 und 40 ° C, und bei einem pH-Wert zwischen 7 und 10, bevorzugt zwischen 8 und 9, wobei man im Falle, daß in der Verbindung der Formeln (23) X ein Fluoratom ist, bevorzugt die Umsetzung bei einer Temperatur zwischen 5 und 15 ° C durchführt.

Ebenfalls erfolgt die Umsetzung einer Verbindung der allgemeinen Formel (24) mit einer Verbindung der allgemeinen Formeln (20) bei einer Temperatur zwischen 10 und 40 ° C, bevorzugt zwischen 30 und 40 ° C, und bei einem pH-Wert zwischen 3 und 8, bevorzugt zwischen 4 und 6, wobei im Falle des Einsatzes einer Verbindung der allgemeinen Formel (24) mit X gleich Fluor bevorzugt die Umsetzung bei einer Temperatur zwischen 5 und 15 ° C durchgeführt wird.

Die Ausgangsverbindungen der allgemeinen Formeln (20c) und (20d) lassen sich, ausgehend von den Verbindungen (20), in an und für sich bekannter Verfahrensweise der Umsetzung von halogensubstituierten Triazinen mit aminogruppenhaltigen Verbindungen herstellen, wie dies beispielsweise oben für die erfindungsgemäßen Verfahrensweisen beschrieben ist, so ebenfalls in wäßrigem oder wäßrig-organischem Medium in der Regel bei einer Temperatur zwischen 0 und 10 ° C, bevorzugt zwischen 0 und 5 ° C, und bei einem pH-Wert zwischen 2 und 7, bevorzugt zwischen 3 und 6, wobei man im Falle einer Ausgangsverbindung der Formel (21) mit X gleich Fluor bevorzugt eine Reaktionstemperatur zwischen -5 ° C und + 5 ° C wählt. In gleicher Weise und unter den gleichen Verfahrensbedingungen lassen sich die Ausgangsverbindungen der allgemeinen Formel (24) durch Umsetzung einer Verbindung der allgemeinen Formel (21) mit einer Verbindung der allgemeinen Formel (22) herstellen.

Die Ausgangsverbindungen der allgemeinen Formeln (20) sind allgemein bekannt und in der Literatur, wie beispielsweise in den anfangs erwähnten Druckschriften, zahlreich beschrieben. Ebenso sind die Ausgangsverbindungen der allgemeinen Formel (22) aus der anfangs erwähnten Literatur bekannt.

Aromatische Amine, die als Diazokomponenten zur Synthese der erfindungsgemäßen Azofarbstoffe der allgemeinen Formeln (1) dienen und der allgemeinen Formeln $R^G$-D-$NH_2$ bzw $R^G$-$D^1$-$NH_2$ entsprechen, sind beispielsweise:

2-(β-Sulfatoethylsulfonyl)-anilin, 3-(β-Sulfatoethylsulfonyl)-anilin, 4-(β-Sulfatoethylsulfonyl)-anilin, 2-Carboxy-5-(β-sulfatoethylsulfonyl)-anilin, 2-Chlor-3-(sulfatoethylsulfonyl)-anilin, 2-Chlor-4-(β-sulfatoethylsulfonyl)-anilin, 2-Brom-4-(β-sulfatoethylsulfonyl)-anilin, 4-Methoxy-3-(β-sulfatoethylsulfonyl)-anilin, 4-Chlor-3-(β-sulfatoethylsulfonyl)-anilin, 2-Ethoxy-4- oder -5-(β-sulfatoethylsulfonyl)-anilin, 2-Methyl-4-(β-sulfatoethylsulfonyl)-anilin, 2-Methoxy-5- oder -4-(β-sulfatoethylsulfonyl]-anilin, 2,4-Diethoxy-5-(β-sulfatoethylsulfonyl)-anilin, 2,4-Dimethoxy-5-(β-sulfatoethylsulfonyl)-anilin, 2,5-Dimethoxy-4-(β-sulfatoethylsulfonyl)-anilin, 2-Methoxy-5-methyl-4-(β-sulfatoethylsulfonyl)-anilin, 2- oder 3- oder 4-(β-Thiosulfatoethylsulfonyl)-anilin, 2-Methoxy-5-(β-thiosulfatoethylsulfonyl)-anilin, 2-Sulfo-4-(β-phosphatoethylsulfonyl)-anilin, 2-Sulfo-4-vinylsulfonyl-anilin, 2-Hydroxy-4- oder -5-(β-sulfatoethylsulfonyl)-anilin, 2-Chlor-4- oder -5-(β-chlorethylsulfonyl)-anilin, 2-Hydroxy-5-(β-sulfatoethylsulfonyl)-anilin, 2-Hydroxy-3-sulfo-5-(β-sulfatoethylsulfonyl)-anilin, 3- oder 4-(β-Acetoxyethylsulfonyl)-anilin, 5-(β-Sulfatoethylsulfonyl)-2-aminonaphthalin, 6- oder 7- oder 8-(β-Sulfatoethylsulfonyl)-2-aminonaphthalin, 6-(β-Sulfatoethylsulfonyl)-1-sulfo-2-aminonaphthalin, 5-(β-Sulfatoethylsulfonyl)-1-sulfo-2-aminonaphthalin, 8-(β-Sulfatoethylsulfonyl)-6-sulfo-2-aminonaphthalin, 4-[β-(β'-Sulfatoethylsulfonyl)-ethyl]-anilin, 3- oder 4-[β-(β'-Chlorethylsulfonyl)-ethylamino]-anilin, 3- oder 4-[β-(β'-Sulfatoethylsulfonyl)-ethylamino]-anilin, 3- oder 4-[γ-(β'-Chlorethylsulfonyl)-propylamino]-anilin, 3- oder 4-[γ-(β'-Sulfatoethylsulfonyl)-propylamino]-anilin und 3- oder 4-[γ-(Vinylsulfonyl)-propylamino]-anilin.

Ausgangsverbindungen entsprechend der allgemeinen Formel $H_2N$-D-$NH_2$ bzw. $H_2N$-$D^2$-$NH_2$ sind beispielsweise 1,4-Phenylen-diamin, 1,4-Phenylendiamin-2-sulfonsäure, 1,4-Phenylendiamin-2-carbonsäure-1,4-Diamino-naphthalin-2-sulfonsäure, 2,6-Diamino-naphthalin-8-sulfonsäure, 2,6-Diamino-naphthalin-4,8-disulfonsäure, 1,3-Phenylen-diamin, 1,3-Phenylendiamin-4-sulfonsäure, 1,3-Phenylen-diamin-4,6-disulfonsäure, 1,4-Phenylendiamin-2,6-disulfonsäure, 1,4-Phenylendiamin-2,5-disulfonsäure, 1,4-Diamino-naphthalin-6-sulfonsäure, 4,4'-Diamino-diphenyl-3-sulfonsäure und 4,4'-Diamino-stilben-2,2'-disulfonsäure.

Ausgangsverbindungen, die zur Herstellung von erfindungsgemäßen Disazofarbstoffen der allgemeinen Formel (1) zunächst als Kupplungskomponente und sodann, in Form der gebildeten Amino-Azoverbindung, als Diazokomponente dienen und der allgemeinen Formel H-E-$NH_2$ entsprechen, sind beispielsweise Anilin, 3-Methyl-anilin, 2-Methoxy-5-methyl-anilin, 2,5-Dimethyl-anilin, 3-Ureido-anilin, 3-Acetylamino-anilin, 3-Propionylamino-anilin, 3-Butyrylamino-anilin, 3-Methoxy-anilin, 2-Methyl-5-acetylamino-anilin, 2-Methoxy-5-acetylamino-anilin, 2-Methoxy-5-methyl-anilin, 3-(Hydroxyacetylamino)-anilin, 1,3-Diaminobenzol-4-sulfonsäure, 1-Aminonaphthalin-6-, -7- oder -8-sulfonsäure 1-Amino-2-methoxy-naphthalin-6-sulfonsäure, 2-Amino-5-hydroxy-naphthalin-7-sulfonsäure, 2-Amino-5-hydroxy-naphthalin-1,7-disulfonsäure, 2-Amino-8-hydroxy-naphthalin-6-sulfonsäure, 2-(4'-Amino-benzoylamino)-5-naphthol-7-sulfonsäure, 1-(4'-Amino-2'-sulfo-phenyl)-3-methyl-5-pyrazolon, 1-(4'-Amino-2'-sulfo-phenyl)-3-carboxy-5-pyrazolon und N-(Acetoacetyl)-3-sulfo-4-amino-anilid.

Ausgangsverbindungen zur Herstellung der erfindungsgemäßen Azoverbindungen der allgemeinen Formel (1), die als Kupplungskomponenten dienen können und den allgemeinen Formeln H-K-$NH_2$ bzw. H-$K^2$-$NH_2$ entsprechen, sind beispielsweise:

1-Amino-3-methylbenzol, 1-Amino-2-methoxy-5-methylbenzol, 1-Amino-2,5-dimethylbenzol, 3-Aminophenylharnstoff, 1-Amino-3-acetylaminobenzol, 1-Amino-3-hydroxyacetylaminobenzol, 1,3-Diaminobenzol-4-sulfonsäure, 1-Amino-2-carboxybenzol-4-sulfonsäure, 1-Amino-4-carboxybenzol-2-sulfonsäure, 1-Amino-4- oder -5-chlorbenzol-2-sulfonsäure, 1-Amino-6-chlorbenzol-3- oder -4-sulfonsäure, 1-Amino-3,4-dichlorbenzol-6-sulfonsäure, 1-Amino-2,5-dichlorbenzol-6-sulfonsäure, 1-Amino-2,5-dichlorbenzol-4-sulfonsäure, 1-Amino-4-methyl-5-chlorbenzol-2-sulfonsäure, 1-Amino-5-methyl-4-chlorbenzol-2-sulfonsäure, 1-Amino-4- oder -5-methoxybenzol-2-sulfonsäure, 1-Amino-6-methoxybenzol-3- oder -4-sulfonsäure, 1-Amino-6-ethoxybenzol-3- oder -4-sulfonsäure, 1-Amino-2,4-dimethoxybenzol-6-sulfonsäure, 1-Amino-2,5-dimethoxybenzol-4-sulfonsäure, 1-Amino-3-acetylaminobenzol-6-sulfonsäure, 2-Amino-1-methylbenzol-3,5-disulfonsäure, 1-Amino-3-nitrobenzol-6-sulfonsäure, 1-Aminonaphthalin, 2-Aminonaphthalin, 1-Aminonaphthalin-2-, -4-, -5-, -6-, -7- oder -8-sulfonsäure, 2-Aminonaphthalin-3,6- oder -5,7-disulfonsäure, 1-Aminonaphthalin-3,6- oder -5,7-disulfonsäure, 2-Aminonaphthalin-1-sulfonsäure, 2-Aminonaphthalin-1,5-, -1,7-, -3,6-, -5,7-, -4,8- oder -6,8-disulfonsäure, 1-Aminonaphthalin-2,5,7-trisulfonsäure, 2-Aminonaphthalin-1,5,7-, 3,6,8- oder -4,6,8-trisulfonsäure, 1-Amino-2-methoxynaphthalin-6-sulfonsäure, 2-Aminonaphthalin-5,7-disulfonsäure, 1-Amino-8-hydroxynaphthalin-6-sulfonsäure, 1-Amino-8-hydroxynaphthalin-2,4-disulfonsäure, 2-Hydroxy-3-aminonaphthalin-5,7-disulfonsäure, 1-Amino-8-hydroxynaphthalin-2,4,6-trisulfonsäure und 2-Amino-5-hydroxynaphthalin-7-sulfonsäure.

Ausgangsverbindungen zur Herstellung von erfindungsgemäßen Azofarbstoffe, die den allgemeinen Formeln H-K-$R^G$ bzw. H-$K^1$-$R^G$ entsprechen, sind beispielsweise:

1-[4'-(β-Sulfatoethyl-sulfonyl]-phenyl]-3-methyl-5-pyrazolon, 1-[4'-(Vinylsulfonyl)-phenyl]-3-methyl-5-pyrazolon, 1-[4'-(β-Sulfatoethylsulfonyl)-phenyl]-3-carboxy-5-pyrazolon, 1-[3'-[β-Chlorethylsulfonyl)-benzoylamino]-3,6-disulfo-8-naphthol, 1-[3'-(Vinylsulfonyl)-benzoylamino]-3,6-disulfo-8-naphthol, 1-[3'-(Vinylsulfonyl)-benzoylamino]-4,6-disulfo-8-naphthol, 1-[3'-(β-Sulfatoethylsulfonyl)-benzoylamino]-4,6-disulfo-8-naphthol, 2-[3'-(β-Chlorethylsulfonyl]-benzoylamino]-6-sulfo-8-naphthol, 2-[3'-(Vinylsulfonyl)-benzoylamino]-6-sulfo-8-naphthol, 3-[3'-(β-Chlorethylsulfonyl)-benzoylamino]-6-sulfo-8-naphthol, 3-[3'-(Vinylsulfonyl)-benzoyl-amino]-6-sulfo-8-naphthol, 6-Sulfo-1-[3'-(β-chlorethylsulfonyl)-benzoylamino]-naphthol, 7-Sulfo-[3'-(vinylsulfonyl)-benzoylamino]-naphthol, 1-[N'-(3'-β-Chlorethylsulfonyl-phenyl)-ureido]-3,6-disulfo-8-naphthol, 1-[N'-(3'-Vinylsulfonyl-phenyl)-ureido]-3,6-disulfo-8-naphthol, 1-[N'-(3'-Vinylsulfonyl-propyl)-ureido]-3,6-disulfo-8-naphthol, 1-[N'-(3'-β-Chlorethylsulfonyl-phenyl)-ureido]-4,6-disulfo-8-naphthol, 1-[N'-(3'-Vinylsulfonyl-phenyl)-ureido]-4,6-disulfo-8-naphthol, 1-[N'-(3'-β-Chlorethylsulfonyl-propyl)-ureido]-4,6-disulfo-8-naphthol, 2-[N'-(3'-β-Sulfatoethylsulfonyl-phenyl)-ureido]-6-sulfo-8-naphthol, 2-[N'-(3'-Chlorethylsulfonyl-propyl)-ureido]-6-sulfo-8-naphthol, 3-[N'-(3'-β-Chlorethylsulfonyl-phenyl)-ureido]-6-sulfo-8-naphthol, 3-[N'-(3'-Vinylsulfonyl-propyl)-ureido]-6-sulfo-8-naphthol, 2-Sulfo-5-[N'-(3''-β-chlorethylsulfonyl)-phenyl]-ureido-anilin, 3-[N'-(3''-β-Sulfatoethylsulfonyl)-phenyl]-ureido-anilin und 6-Sulfo-1-[N'-(3''-β-sulfatoethylsulfonyl)-phenyl]-ureido-8-naphthol.

Geht man von Diazokomponenten der Formel $H_2N$-D-$NH_2$ bzw. $H_2N$-$D^2$-$NH_2$ aus, so können diese auch in der Form der Monoacylamino-amino-Verbindungen eingesetzt werden, wobei der Acylrest insbesondere der Acetylrest ist. Diese Monoacylamino-amino-Verbindungen werden zunächst diazotiert und mit einer kuppelfähigen Verbindungn gekuppelt; anschließend wird der Acylrest hydrolytisch abgespalten, und die so nunmehr wieder frei gewordene Aminogruppe kann mit dem faserreaktiven Rest $Z^1$ verbunden werden. Solche monoacylierten Diamine sind beispielsweise 2-Sulfo-5-acetylamino-anilin und 2-Sulfo-4-acetylamino-anilin. In gleicher Weise können aminogruppenhaltige Kupplungskomponenten in Form des Acylamino-Derivates in die Kupplungsreaktion eingesetzt werden, wobei anschließend auch hier der Acylrest hydrolytisch abgespalten werden kann, um die freiwerdende Aminogruppe mit der faserreaktiven Gruppe $Z^1$ zu verbinden.

Bivalente Kupplungskomponenten, die zum Aufbau erfindungsgemäßer Disazofarbstoffe dienen können, in welchen der bivalente Kupplungsrest mit zwei Diazokomponenten verbunden ist, von denen die eine oder beide einen faserreaktiven Rest Z enthalten, beispielsweise von Farbstoffen der allgemeinen Formel (3d), sind beispielsweise Resorcin, 1,3-Diaminobenzol, 5,5'-Dihydroxy-7,7'-disulfo-2,2'-dinaphthyl-harnstoff, 1,8-Dihydroxy-3,6-disulfo-naphthalin und insbesondere 1-Amino-8-naphthol-3,6-disulfonsäure und 1-Amino-8-naphthol-4,6-disulfonsäure.

Geht man bei der erfindungsgemäßen Synthese der Azofarbstoffe von Diazo- oder Kupplungskomponenten aus, die bereits die Gruppe der allgemeinen Formel (2) enthalten, erfolgen die Umsetzungen in der üblichen Verfahrensweise der Diazotierungs- und Kupplungsreaktionen, so die Diazotierung in der Regel bei einer Temperatur zwischen -5 °C und + 15 °C und einem pH-Wert unterhalb von 2 mittels einer starken Säure und Alkalinitrit in bevorzugt wäßrigem Medium und die Kupplungsreaktion in der Regel bei einem pH-Wert zwischen 1,5 und 4,5 im Falle einer aminogruppenhaltigen Kupplungskomponente und bei einem pH-Wert zwischen 3 und 7,5 im Falle einer hydroxygruppenhaltigen Kupplungskomponente und bei einer Temperatur zwischen 0 und 25 °C, ebenso bevorzugt in wäßrigem Medium.

Die erfindungsgemäßen Azofarbstoffe der allgemeinen Formel (1) - im nachfolgenden als Verbindungen (1) bezeichnet - haben faserreaktive Eigenschaften und besitzen sehr wertvolle Farbstoffeigenschaften. Sie können deshalb zum Färben (einschließlich Bedrucken) von hydroxygruppenhaltigen und/oder carbonamidgruppenhaltigen Materialien verwendet werden. Hierzu können die bei der Synthese der Verbindungen (1) anfallenden Lösungen, gegebenenfalls nach Zusatz einer Puffersubstanz und gegebenenfalls auch nach Konzentrierung, direkt als Flüssigpräparation der färberischen Verwendung zugeführt werden.

Die Abscheidung und Isolierung der Verbindungen (1) aus den wäßrigen Syntheselösungen kann nach allgemein bekannten Methoden für wasserlösliche Verbindungen erfolgen, so beispielsweise durch Ausfällen aus dem Reaktionsmedium mittels eines Elektrolyten, wie beispielsweise Natriumchlorid oder Kaliumchlorid, oder aber duch Eindampfen der Reaktionslösung selbst, beispielsweise durch Sprühtrocknung. Falls die letztgenannte Art der Isolierung gewählt wird, empfiehlt es sich vielfach, vor dem Eindampfen eventuell in den Lösungen vorhandene Sulfatmengen durch Fällung als Calciumsulfat und Abtrennung mittels Filtration zu entfernen.

Gegenstand der vorliegenden Erfindung ist deshalb auch die Verwendung der Verbindungen (1) zum Färben (einschließlich Bedrucken) von hydroxy- und/oder carbonamidgruppenhaltigen Materialien bzw. Verfahren zu deren Anwendung auf diesen Substraten. Bevorzugt kommen die Materialien in Form von Fasermaterialien zur Anwendung, insbesondere in Form von Textilfasern, wie Garnen, Wickelkörpern und Geweben. Hierbei kann man analog bekannten Verfahrensweisen vorgehen.

Hydroxygruppenhaltige Materialien sind solche natürlichen oder synthetischen Ursprungs, wie beispielsweise Cellulosefasermaterialien oder deren Regenerationsprodukte und Polyvinylalkohole. Cellulosefasermaterialien sind vorzugsweise Baumwolle, aber auch andere Pflanzenfasern, wie Leinen, Hanf, Jute und Ramiefasern; regenerierte Cellulosefasern sind beispielsweise Zellwolle und Viskosekunstseide.

Carbonamidgruppenhaltige Materialien sind beispielsweise synthetische und natürliche Polyamide und Polyurethane, insbesondere in Form von Fasern, beispielsweise Wolle und andere Tierhaare, Seide, Leder, Polyamid-6,6, Polyamid-6, Polyamid-11 und Polyamid-4.

Die Verbindungen (1) lassen sich, gemäß der erfindungsgemäßen Anwendung, auf den genannten Substraten, insbesondere auf den genannten Fasermaterialien, nach den für wasserlösliche, faserreaktive Farbstoffe bekannten Anwendungstechniken applizierten und fixieren, so beispielsweise, indem man die Verbindung (1) in gelöster Form auf das Substrat aufbringt oder sie darin einbringt und sie auf diesem oder

in diesem durch Hitzeeinwirkung oder durch Einwirkung eines alkalisch wirkenden Mittels oder durch beide Maßnahmen fixiert. Solche Färbe- und Fixierweisen sind in der Literatur zahlreich beschrieben (bspw. in der europäischen Patentanmeldungs-Veröffentlichung Nr. 0 181 585 A2).

Die erfindungsgemäß erhältlichen Färbungen besitzen, insbesondere auf Cellulosefasermaterialien, gute Lichtechtheiten sowohl im trockenen Zustand der Färbung als auch im nassen, beispielsweise mit einer Schweißlösung befeuchteten, Zustand sowie gute Naßechtheiten, wie beispielsweise gute Waschechtheiten bei 60 bis 95 °C, auch in Gegenwart von Perboraten, gute saure und alkalische Walk-, Überfärbe- und Schweißechtheiten, eine hohe Dampfbeständigkeit, gute Alkali-, Säure-, Wasser- und Seewasserechtheiten sowie gute Plissier-, Bügel- und Reibechtheiten. Ebenso besitzen sie eine gute Säurelagerbeständigkeit ("acid fading") beim Lagern von feuchten, noch Essigsäure enthaltendem, gefärbtem Material.

Die nachstehenden Beispiele dienen zur Erläuterung der Erfindung. Die Teile sind Gewichtsteile, die Prozentangaben stellen Gewichtsprozente dar, sofern nicht anders vermerkt. Gewichtsteile beziehen sich zu Volumenteilen wie Kilogramm zu Liter.

Die in den Beispielen formelmäßig beschriebenen Verbindungen sind in Form der freien Säuren angegeben; im allgemeinen werden sie in Form ihrer Alkalimetallsalze, wie Lithium-, Natrium- oder Kaliumsalze, hergestellt und isoliert und in Form ihrer Salze zum Färben verwendet. Ebenso können die in den nachfolgenden Beispielen, insbesondere Tabellenbeispielen, in Form der freien Säure genannten Ausgangsverbindungen und Komponenten als solche oder in Form ihrer Salze, vorzugsweise Alkalimetallsalze, in die Synthese eingesetzt werden.

Die für die erfindungsgemäßen Verbindungen angegebenen Absorptionsmaxima ($\lambda_{max}$) im sichtbaren Bereich wurden anhand der Alkalimetallsalze in wäßriger Lösung ermittelt. In den Tabellenbeispielen sind die $\lambda_{max}$-Werte bei der Farbtonangabe in Klammern gesetzt; die Wellenlängeangabe bezieht sich auf nm.

Beispiel 1

a) 127,6 Teile 1-Amino-8-naphthol-3,6-disulfonsäure werden unter Zugabe von 16,8 Teilen Natriumfluorid und der zur Einstellung eines pH-Wertes zwischen 6,5 und 7 erforderlichen Menge an konzentrierter wäßriger Natronlauge in 2000 Teilen Wasser gelöst und in einem kontinuierlichen Verfahren mit 55 Teilen Cyanurfluorid (2,4,6-Trifluor-1,3,5-triazin) umgesetzt. Die erhaltene Lösung gibt man zu einer Suspension von 156 Teilen Bis-[γ-(β'-chlorethylsulfonyl)-propyl]-amin-Hydrochlorid in 500 Teilen Eiswasser und hält den pH-Wert mittels einer wäßrigen Natriumcarbonatlösung zwischen 8 und 10. Man rührt noch etwa 60 Minuten bei 0 bis 5 °C weiter, stellt den pH-Wert sodann auf 6 und isoliert das so hergestellte Gemisch der Verbindungen 1-[2'-Bis-(vinylsulfonyl-propyl)-amino-4'-fluor-1',3',5'-triazin-6'-yl]-amino-8-naphthol-3,6-disulfonsäure und 1-{2'-Bis-[γ-(β'-chlorethylsulfonyl)-propyl]-amino-4'-fluor-1',3',5'-triazin-6'-yl}-amino-8-naphthol-3,6-disufonsäure und deren Bis-(γ-vinylsulfonyl)-Abkömmlinge in Form der Natriumsalze durch Aussalzen mit Natriumchlorid.

b) 76,8 Teile der unter a) hergestellten Verbindungen werden in 500 Teilen Eiswasser suspendiert und langsam unter Rühren mit der auf üblichem Wege hergestellten salzsauren wäßrigen Suspension des Diazoniumsalzes von 39,5 Teilen 1-Sulfo-6-(β-sulfatoethylsulfonyl)-2-amino-naphthalin unter Einhaltung eines pH-Wertes von 6 mittels wäßriger Natriumcarbonatlösung versetzt. Nach Beendigung der Kupplungsreaktion wird das Gemisch der beiden erfindungsgemäßen Azofarbstoffe, die, in Form der freien Säure geschrieben, die Formel

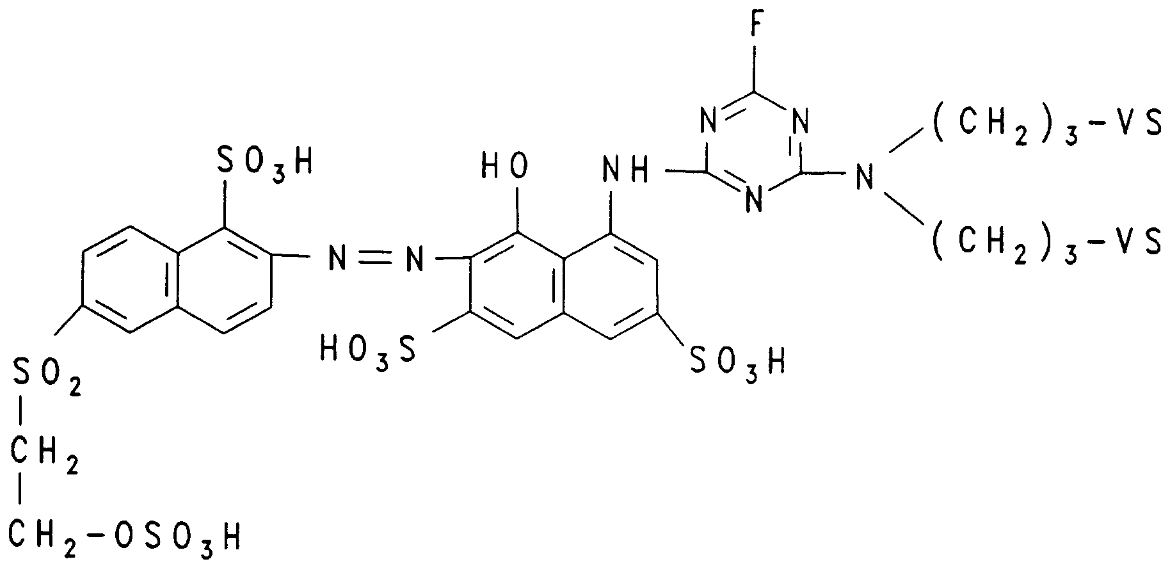

($\lambda_{max}$ = 540 nm)

besitzen, in welcher die beiden Gruppen VS den Vinylsulfonyl- und den $\beta$-Chlorethylsulfonyl-Rest bedeuten, in üblicher Weise durch Aussalzen mit Natriumchlorid isoliert.

Unter Anwendung der in der Technik für faserreaktive Farbstoffe üblichen Applikations- und Fixierverfahren erhält man mit dem Farbstoff auf den in der Beschreibung genannten Fasermaterialien, wie insbesondere Cellulosefasermaterialien, farbstarke, echte rote Färbungen und Drucke.

Beispiel 2

a) 95,6 Teile 3-Amino-8-hydroxy-naphthalin-7-sufonsäure werden unter Zugabe von 9,6 Teilen Lithiumhydroxid in 2500 Teilen Eiswasser gelöst und in einem kontinuierlichen Verfahren mit 55 Teilen Cyanurfluorid umgesetzt. Anschließend gibt man eine Suspension von 156 Teilen Bis-[$\gamma$-($\beta$'-chlorethylsulfonyl)-propyl]-amin-Hydrochlorid in 500 Teilen Eiswasser hinzu und führt die Umsetzung unter Einhaltung eines pH-Wertes zwischen 8 und 10 bei 0 bis 5 °C zu Ende. Man isoliert das Gemisch der Verbindungen der Formel

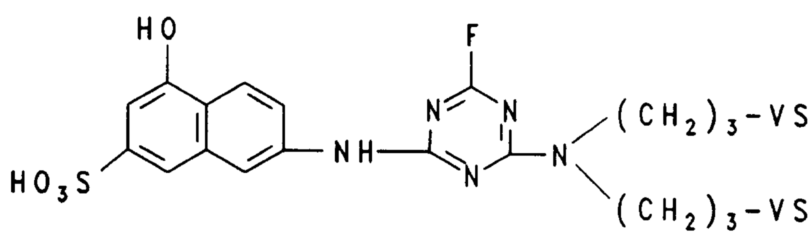

(in welcher VS die Vinylsulfonyl- und die $\beta$-Chlorethylsulfonyl-Gruppebedeutet) in üblicher Weise durch Aussalzen mit Natriumchlorid als Natriumsalz.

b) 69,9 Teile der unter a) hergestellten Kupplungskomponente werden in 500 Teilen Eiswasser suspendiert und langsam unter gutem Rühren mit einer in üblicher Weise hergestellten salzsauren wäßrigen Suspension des Diazoniumsalzes aus 39,5 Teilen 1-Sulfo-6-($\beta$-sulfatoethylsulfonyl)-2-amino-naphthalin unter Einhaltung eines pH-Wertes von 6 versetzt. Man rührt bei diesem pH-Wert noch einige Zeit bis zur Beendigung der Kupplungsreaktion nach und isoliert sodann die erfindungsgemäße Azoverbindung (als Gemisch der Vinylsulfonyl- und $\beta$-Chlorethylsulfonyl-Derivate) entsprechend der Formel (in Form der freien Säure geschrieben)

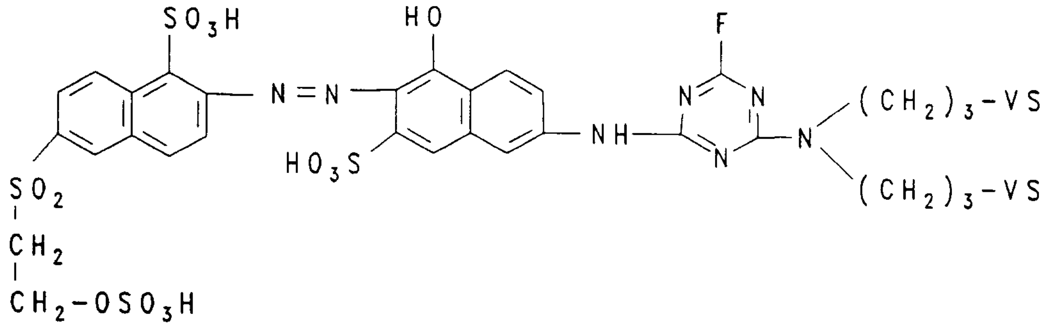

($\lambda_{max}$ = 480 nm)

als Alkalimetallsalz (Natriumsalz) durch Aussalzen mit Natriumchlorid.

Der erfindungsgemäßen Azofarbstoff färbt nach den in der Technik für faserreaktive Farbstoffe üblichen Anwendungsverfahren beispielsweise Cellulosefasermaterialien, wie Baumwolle, jedoch ebenso Wolle und synthetische Polyamidfasern, in farbstarken, brillanten orangen Tönen mit guten Echtheitseigenschaften.

Beispiel 3

18,8 Teile 1,3-Diaminobenzol-4-sulfonsäure und 18,4 Teile 2,4,6-Trichlor-1,3,5-triazin (Cyanurchlorid) werden in 300 Teilen Eiswasser suspendiert; während der etwa dreistündigen Umsetzung wird mittels wäßriger Natriumcarbonatlösung ein pH-Wert zwischen 1,5 und 2 gehalten. Anschließend gibt man 39 Teile Bis-[γ-(β'-chlorethylsulfonyl)-propyl]-amin-Hydrochlorid hinzu und führt die Umsetzung bei 40 °C und bei einem pH-Wert von 8 durch.

Zu der so hergestellten Kupplungskomponente gibt man die auf üblichem Wege hergestellte schwefelsaure wäßrige Suspension des Diazoniumsalzes aus 28 Teilen 4-(β-Sulfatoethylsulfonyl)-anilin und führt die Kupplungsreaktion bei einem pH-Wert von 5 und einer Temperatur zwischen 10 und 15 °C durch. Der erhaltene erfindungsgemäße Azofarbstoff wird als Gemisch seiner Vinylsulfonyl- und β-Chlorethylsulfonyl-Derivate aus der Syntheselösung durch Aussalzen mit Natriumchlorid als Alkalimetallsalz isoliert. Der Farbstoff entspricht, in Form der freien Säure geschrieben, der Formel

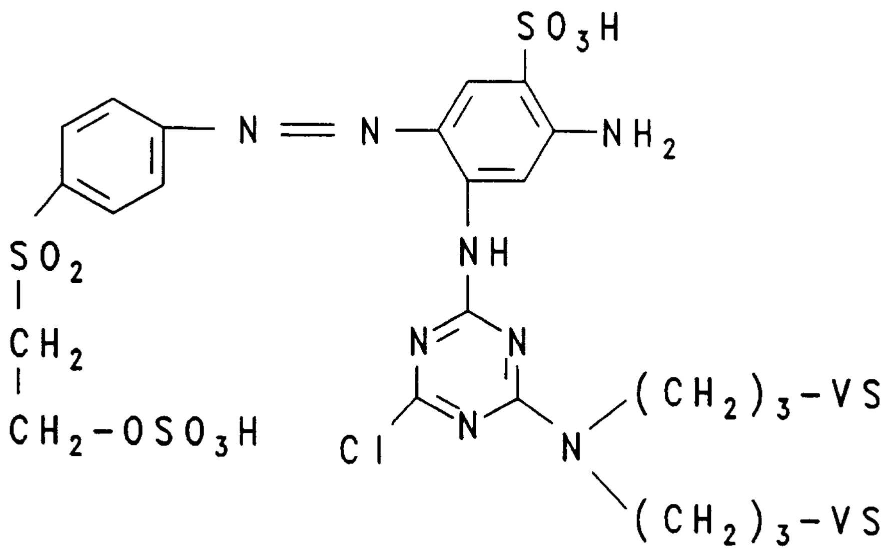

($\lambda_{max}$ = 413 nm)

in welcher VS für die Vinylsulfonyl- und die β-Chlorethylsulfonyl-Gruppe steht.

Er besitzt sehr gute faserreaktive Farbstoffeigenschaften und liefert auf den in der Beschreibung genannten Materialien, wie insbesondere Cellulosefasermaterialien, nach den für faserreaktive Farbstoffe üblichen Färbe- und Druckverfahren farbstarke, goldgelbe Färbungen und Drucke mit guten Echtheitseigenschaften.

Beispiel 4

Man suspendiert 18,8 Teile 1,3-Diaminobenzol-4-sulfonsäure in 300 Teilen Eiswasser und gibt unter kräftigem Rühren 13,5 Teile Cyanurfluorid hinzu. Man rührt noch einige Zeit nach und versetzt den Ansatz sodann mit 39 Teilen Bis-[γ-(β'-chlorethylsulfonyl)-propyl]-amin-Hydrochlorid und führt die Umsetzung bei einer Temperatur von etwa 5 °C und bei einem pH-Wert von 9 durch.

Zu der so hergestellten Lösung der Kupplungskomponente gibt man eine auf üblichem Wege hergestellte schwefelsaure wäßrige Suspension des Diazoniumsalzes von 28 Teilen 4-(β-Sulfatoethylsulfonyl)-anilin und führt die Kupplungsreaktion bei einem pH-Wert von 5 und einer Temperatur zwischen 10 und 15 °C durch. Man isoliert das Gemisch der erfindungsgemäßen Farbstoffe, die, in Form der freien Säure, die Formel

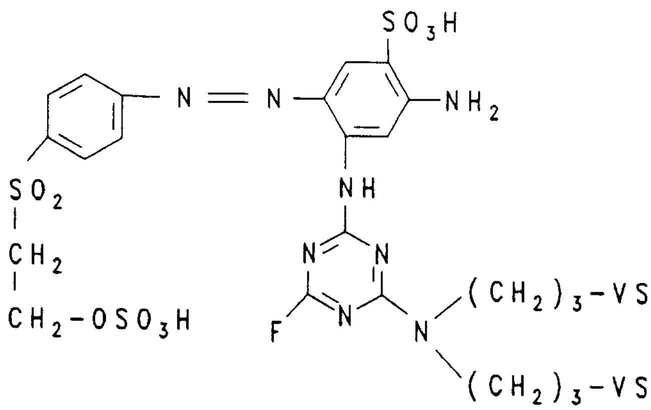

($\lambda_{max}$ = 411 nm)

mit VS gleich den Vinylsulfonyl- und β-Chlorsulfonyl-Gruppen besitzen, in üblicher Weise durch Aussalzen mit Natriumchlorid. Der Farbstoff zeigt sehr gute faserreaktive Farbstoffeigenschaften und färbt beispielsweise Baumwolle nach den in der Technik für faserreaktive Farbstoffe üblichen Applikations- und Fixierverfahren in farbstarken, echten goldgelben Tönen.

Beispiel 5

31 Teile 1-Amino-8-hydroxy-naphthalin-3,6-disulfonsäure und 18,4 Teile Cyanurchlorid werden in 500 Teilen Eiswasser suspendiert; die Umsetzung erfolgt bei einer Temperatur von etwa 5 °C und einem pH-Wert zwischen 2 und 2,5. Nach Beendigung der Kondensationsreaktion gibt man zu diesem Reaktionsansatz eine auf üblichem Wege hergestellte schwefelsaure wäßrige Suspension des Diazoniumsalzes von 28 Teilen 4-(β-Sulfatoethylsulfonyl)-anilin und führt die Kupplungsreaktion bei 5 bis 10 °C und unter Einhaltung eines pH-Wertes zwischen 4 und 5 durch.

Die so hergestellte Dichlor-triazinylamino-naphthol-Azoverbindung wird mit 39 Teilen Bis-[γ-(β-chlorethylsulfonyl)-propyl]-amin-Hydrochlorid versetzt; der Ansatz wird auf 50 °C erwärmt und ein pH-Wert von 8 eingestellt; die Reaktionsbedingungen werden etwa drei Stunden bis zur Beendigung der zweiten Kondensationsreaktion gehalten. Danach wird der Ansatz auf 25 °C abgekühlt und der pH-Wert auf 9 bis 10 erhöht und während vier Stunden gehalten, wobei sowohl die β-Chlorethylsulfonyl-Gruppen als auch die β-Sulfatoethylsulfonyl-Gruppe in die Vinylsulfonylgruppen übergeführt werden.

Der erfindungsgemäße Azofarbstoff, der, in Form der freien Säure geschrieben, die Formel

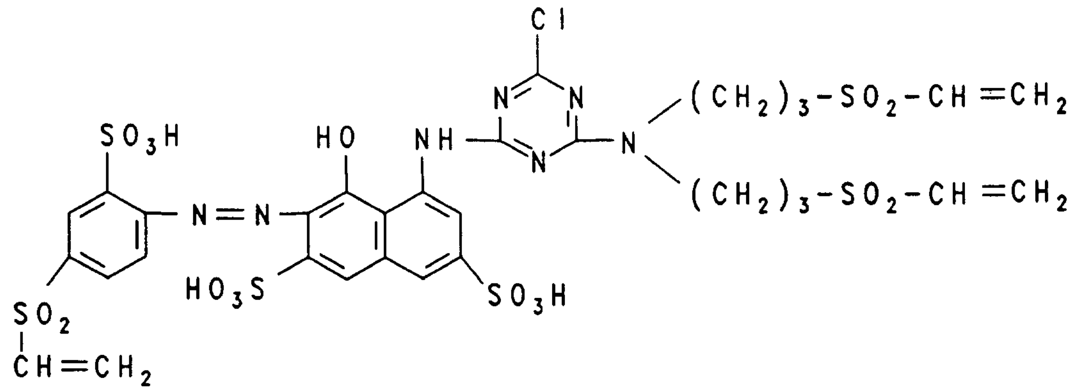

($\lambda_{max}$ = 522 nm)
besitzt, wird in üblicher Weise isoliert. Er färbt die in der Beschreibung genannten Materialien, wie insbesondere Cellulosefasermaterialien, in farbstarken roten, echten Tönen.

Beispiele 6 bis 66

In den nachfolgenden Tabellenbeispielen sind weitere erfindungsgemäße Azofarbstoffe entsprechend der allgemeinen Formel (A)

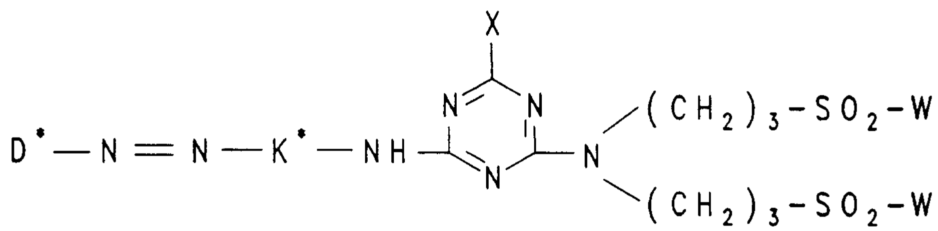

(A)

mit Hilfe ihrer Komponenten beschrieben. Sie lassen sich nach einer der erfindungsgemäßen Herstellungsweisen, beispielsweise analog einem der obigen Beispiele, unter Einsatz der aus dem jeweiligen Tabellenbeispiel in Verbindung mit der allgemeinen Formel (A) ersichtlichen Ausgangsverbindungen (wie der Diazokomponente D*-$NH_2$, der kupplungsfähigen Verbindung H-K*-$NH_2$, Cyanurchlorid oder Cyanurfluorid und einer Verbindung entsprechend der allgemeinen Formel (22)) herstellen. Sie besitzen sehr gute faserreaktive Farbstoffeigenschaften und färben die in der Beschreibung genannten Materialien, insbesondere Cellulosefasermaterialien, wie Baumwolle, in dem in dem jeweiligen Tabellenbeispiel (hier für Baumwolle) angegebenen Farbton in hoher Farbstärke und mit guten Echtheiten.
Sofern in der Tabelle der Rest W mit VS bezeichnet ist, zeigt die Gruppierung VS an, daß der erfindungsgemäße Azofarbstoff als Gemisch der Vinylsulfonyl- und $\beta$-Chlorethylsulfonyl-Derivate vorliegt.

| Bsp. | Rest D*- | Rest -K*-NH- | Rest X | Rest W | Farbton |
|---|---|---|---|---|---|
| 6 | 3-Vinylsulfonyl-phenyl | 3,6-Disulfo-8-hydroxy-naphth-7,1-ylen-1-amino | Chlor | Vinyl | rot (520) |
| 7 | 1-Sulfo-6-(ß-sulfatoethylsulfonyl)-naphth-2-yl | 4,6-Disulfo-8-hydroxy-naphth-7,1-ylen-1-amino | Fluor | VS | rot |
| 8 | 4-(ß-Sulfatoethylsulfonyl)-phenyl | 3,6-Disulfo-8-hydroxy-naphth-7,1-ylen-1-amino | Fluor | VS | rot (523) |
| 9 | 3-Vinylsulfonyl-phenyl | dito | Fluor | VS | rot (521) |
| 10 | 2-Methoxy-5-(ß-sulfatoethylsulfonyl)-phenyl | dito | Fluor | VS | rot (543) |
| 11 | 6-Sulfo-8-(ß-sulfatoethylsulfonyl)-naphth-2-yl | dito | Fluor | VS | rot (534) |
| 12 | 6-(ß-Sulfatoethylsulfonyl)-naphth-2-yl | dito | Fluor | VS | rot |
| 13 | 8-(ß-Sulfatoethylsulfonyl)-naphth-2-yl | dito | Fluor | VS | rot (551) |
| 14 | 2,5-Dimethoxy-4-(ß-sulfatoethyl-sulfonyl)-phenyl | dito | Fluor | VS | rot (541) |

| Bsp. | Rest $D^*$- | Rest -$K^*$-NH- | Rest X | Rest W | Farbton |
|---|---|---|---|---|---|
| 15 | 2-Methoxy-5-methyl-4-(ß-sulfato-ethylsulfonyl)-phenyl | dito | Fluor | VS | rot |
| 16 | 2-Chlor-5-(ß-sulfatoethylsulfonyl)-phenyl | dito | Fluor | VS | rot |
| 17 | 2-Sulfo-4-(ß-sulfatoethylsulfonyl)-phenyl | dito | Fluor | VS | rot (514) |
| 18 | 4-Vinylsulfonyl-phenyl | 6-Sulfo-8-hydroxy-naphth-7,3-ylen-3-amino | Fluor | VS | orangegelb |
| 19 | 1-Sulfo-6-(ß-sulfatoethylsulfonyl)-phenylen | dito | Fluor | VS | orange |
| 20 | 2,5-Dimethyl-4-(ß-sulfatoethyl-sulfonyl)-phenyl | dito | Fluor | VS | orangerot |
| 21 | 3-(ß-Sulfatoethylsulfonyl)-phenyl | dito | Fluor | VS | orange |
| 22 | 2-Methoxy-5-methyl-4-(ß-sulfato-ethylsulfonyl)-phenyl | dito | Fluor | VS | orange |

| Bsp. | Rest $D^*$- | Rest $-K^*$-NH- | Rest X | Rest W | Farbton |
|---|---|---|---|---|---|
| 23 | 4-(ß-Sulfatoethylsulfonyl)-phenyl | dito | Fluor | VS | orange (471) |
| 24 | 3-Vinylsulfonyl-phenyl | dito | Fluor | VS | orange (475) |
| 25 | 2-Methoxy-5-(ß-sulfatoethylsulfonyl)-phenyl | dito | Fluor | VS | orange (490) |
| 26 | 6-Sulfo-8-(ß-sulfatoethylsulfonyl)-naphth-2-yl | dito | Fluor | VS | orange (480) |
| 27 | 6-(ß-Sulfatoethylsulfonyl)-naphth-2-yl | dito | Fluor | VS | orange |
| 28 | 8-(ß-Sulfatoethylsulfonyl)-naphth-2-yl | dito | Fluor | VS | orange (500) |
| 29 | 2,5-Dimethoxy-4-(ß-sulfatoethyl-sulfonyl)-phenyl) | dito | Fluor | VS | orange (489) |
| 30 | 2-Methoxy-5-methyl-4-(ß-sulfato-ethylsulfonyl)-phenyl | dito | Fluor | VS | rotstichig orange |
| 31 | 2-Chlor-5-(ß-sulfatoethylsulfonyl)-phenyl | dito | Fluor | VS | orange |

| Bsp. | Rest $D^*$- | Rest -$K^*$-NH- | Rest X | Rest W | Farbton |
|---|---|---|---|---|---|
| 32 | 2-Sulfo-4-(ß-sulfatoethylsulfonyl)-phenyl | dito | Fluor | VS | orange (461) |
| 33 | 1-Sulfo-6-(ß-sulfatoethylsulfonyl)-naphth-2-yl | 6-Sulfo-8-hydroxy-naphth-7,2-ylen-2-amino | Fluor | VS | orange (510) |
| 34 | 4-(ß-Sulfatoethylsulfonyl)-phenyl | dito | Fluor | VS | rotbraun (498) |
| 35 | 2-Methoxy-5-methyl-4-(ß-sulfato-ethylsulfonyl)-phenyl | 4,6-Disulfo-8-hydroxy-naphth-7,1-ylen-1-amino | Chlor | Vinyl | rot |
| 36 | 2-Methoxy-4-(ß-sulfatoethylsulfonyl)-phenyl | 1-Amino-2-sulfo-phen-4,3-ylen-3-amino | Chlor | VS | goldgelb |
| 37 | dito | dito | Fluor | VS | goldgelb |
| 38 | 2,5-Dimethoxy-4-(ß-sulfatoethyl-sulfonyl)-phenyl | dito | Chlor | VS | goldorange |
| 39 | dito | dito | Fluor | VS | goldorange |
| 40 | 3-(ß-Sulfatoethylsulfonyl)-phenyl | dito | Chlor | VS | goldgelb |

| Bsp. | Rest $D^*$- | Rest -$K^*$-NH- | Rest X | Rest W | Farbton |
|---|---|---|---|---|---|
| 41 | dito | dito | Fluor | VS | goldgelb |
| 42 | 1-Sulfo-6-(ß-sulfatoethylsulfonyl)-naphth-2-yl | dito | Chlor | VS | goldgelb |
| 43 | 2-Chlor-5-(ß-sulfatoethylsulfonyl)-phenyl | dito | Chlor | VS | goldgelb |
| 44 | 2-Methoxy-5-(ß-sulfatoethylsulfonyl)-phenyl | 3,6-Disulfo-8-hydroxy-naphth-7,1-ylen-1-amino | Chlor | Vinyl | rot (543) |
| 45 | 6-Sulfo-8-(ß-sulfatoethylsulfonyl)-naphth-2-yl | dito | Chlor | Vinyl | rot (535) |
| 46 | 6-(ß-Sulfatoethylsulfonyl)-naphth-2-yl | dito | Chlor | Vinyl | rot |
| 47 | 8-(ß-Sulfatoethylsulfonyl)-naphth-2-yl | dito | Chlor | Vinyl | rot (552) |
| 48 | 2,5-Dimethoxy-4-(ß-sulfatoethyl-sulfonyl)-phenyl | dito | Chlor | Vinyl | rot (541) |
| 49 | 2-Methoxy-5-methyl-4-(ß-sulfato-ethylsulfonyl)-phenyl | dito | Chlor | Vinyl | rot |

| Bsp. | Rest $D^*$- | Rest $-K^*$-NH- | Rest X | Rest W | Farbton |
|---|---|---|---|---|---|
| 50 | 2-Chlor-5-(ß-sulfatoethylsulfonyl)-phenyl | dito | Chlor | Vinyl | rot |
| 51 | 2-Sulfo-4-(ß-sulfatoethylsulfonyl)-phenyl | dito | Chlor | Vinyl | rot (513) |
| 52 | 4-Vinylsulfonyl-phenyl | 4,6-Disulfo-8-hydroxy-naphth-7,1-ylen-1-amino | Chlor | Vinyl | rot (522) |
| 53 | 1-Sulfo-6-(ß-sulfatoethylsulfonyl)-phenyl | dito | Chlor | Vinyl | rot (536) |
| 54 | 2,5-Dimethyl-4-(ß-sulfatoethyl-sulfonyl)-phenyl | dito | Chlor | Vinyl | rot (537) |
| 55 | 8-(ß-Sulfatoethylsulfonyl)-naphth-2-yl | dito | Chlor | Vinyl | rot (546) |
| 56 | 1-Sulfo-6-(ß-sulfatoethylsulfonyl)-2-naphth-2-yl | 6-Sulfo-8-hydroxy-naphth-7,3-ylen-3-amino | Chlor | Vinyl | orange (479) |
| 57 | 4-(ß-Sulfatoethylsulfonyl)-phenyl | dito | Chlor | Vinyl | orange (470) |
| 58 | 3-Vinylsulfonyl-phenyl | dito | Chlor | Vinyl | orange (475) |

| Bsp. | Rest D*- | Rest -K*-NH- | Rest X | Rest W | Farbton |
|---|---|---|---|---|---|
| 59 | 2-Methoxy-5-(ß-sulfatoethylsulfonyl)-phenyl | dito | Chlor | Vinyl | orange (489) |
| 60 | 6-Sulfo-8-(ß-sulfatoethylsulfonyl)-naphth-2-yl | dito | Chlor | Vinyl | orange (481) |
| 61 | 6-(ß-Sulfatoethylsulfonyl)-naphth-2-yl | dito | Chlor | Vinyl | orange |
| 62 | 8-(ß-Sulfatoethylsulfonyl)-naphth-2-yl | dito | Chlor | Vinyl | orange (500) |
| 63 | 2,5-Dimethoxy-4-(ß-sulfatoethylsulfonyl)-phenyl | dito | Chlor | Vinyl | orange (490) |
| 64 | 2-Methoxy-5-methyl-4-(ß-sulfatoethylsulfonyl)-phenyl | dito | Chlor | Vinyl | rotstichig orange |
| 65 | 2-Chlor-5-(ß-sulfatoethylsulfonyl)-phenyl | dito | Chlor | Vinyl | orange |
| 66 | 2-Sulfo-4-(ß-sulfatoethylsulfonyl)-phenyl | dito | Chlor | Vinyl | orange (460) |

Beispiel 67

Man setzt gemäß den Angaben des Beispieles 3a) 18,8 Teile 1,3-Diaminobenzol-4-sulfonsäure, 18,4 Teile Trichlortriazin und 39 Teile Bis-[$\gamma$-($\beta$'-chlorethylsulfonyl)-propyl]-amin-Hydrochlorid um. Die so hergestellte Verbindung dient als Diazokomponente zur Herstellung eines erfindungsgemäßen Azofarbstoffes, indem man 65,3 Teile dieser 2-Sulfo-5-{2'-bis-[γ-(β'-chlorethylsulfonyl)-propyl]-amino-4'-chlor-1',3',5'-triazin-6'-yl}-amino-anilin-Verbindung, in 400 Teilen Eiswasser gelöst und vermischt mit 20 Vol.-Teilen einer 5N-Natriumnitritlösung, langsam in ein Gemisch aus 200 Teilen Eiswasser und 30 Vol.-Teilen einer konzentrierten wäßrigen Lösung gibt, diesen Diazotierungsansatz noch drei Stunden nachrührt, sodann die erhaltene Diazoniumsalzsuspension zu einer 10 °C warmen wäßrigen Suspension von 61 Teilen der auf bekanntem Wege hergestellten Azoverbindung 2-[4'-(β-Sulfatoethylsulfonyl)-phenyl]-azo-1-amino-8-hydroxy-naphthalin-3,6-disulfonsäure gibt und die Kupplungsreaktion bei 10 °C und einem pH-Wert von 6 bis 7 durchführt.

Der erfindungsgemäße Disazofarbstoff, der, in Form der freien Säure geschrieben, die Formel

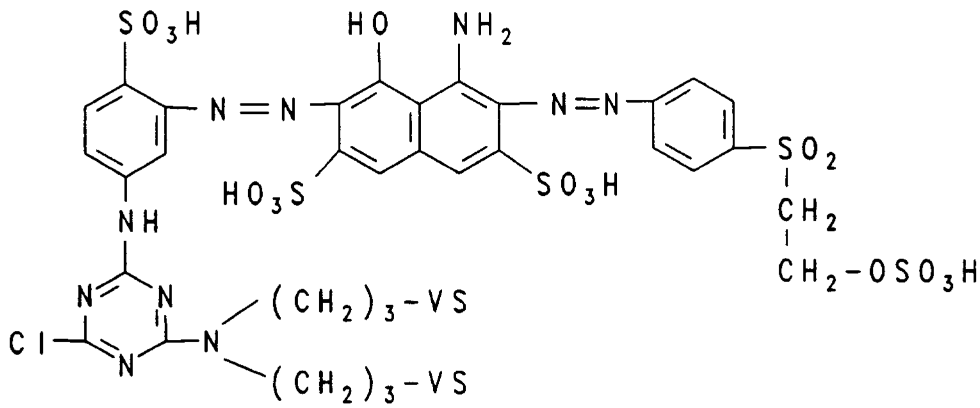

($\lambda_{max}$ = 602 nm)

(in welcher VS für die Vinylsulfonyl- und die β-Chlorethylsulfonyl-Gruppe steht) besitzt, wird in üblicher Weise durch Aussalzen mit Natriumchlorid isoliert. Er zeigt sehr gute Farbstoffeigenschaften und liefert beispielsweise auf Baumwolle nach den in der Technik für faserreaktive Farbstoffe üblichen Färbe- und Druckverfahren Färbungen und Drucke in kräftigen, marineblauen, echten Tönen.

Beispiel 68

Zur Herstellung eines erfindungsgemäßen Disazofarbstoffes verfährt man gemäß den Angaben des Beispieles 6, verwendet jedoch als Diazokomponente die gemäß den Angaben des Beispieles 4 hergestellte 2-Sulfo-5-{2'-bis-[γ-(β'-chlorethylsulfonyl)-propyl]-amino-4'-fluor-1',3',5'-triazin-6'-yl}-amino-anilin-Verbindung.

Der erhaltene erfindungsgemäße Disazofarbstoff, der, in Form der freien Säure geschrieben, der Formel

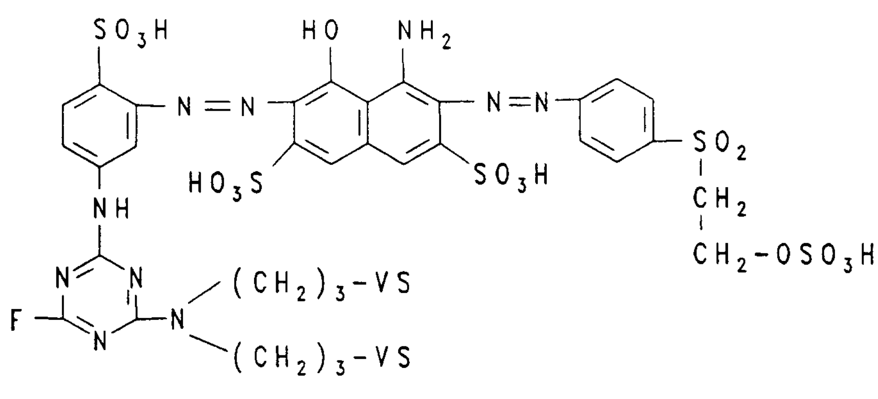

($\lambda_{max}$ = 600)

entspricht (in welcher VS für die Vinylsulfonyl- und die β-Chlorethylsulfonyl-Gruppe steht), zeigt ebenfalls sehr gute Farbstoffeigenschaften und liefert beispielsweise auf Wolle nach den in der Technik für faserreaktive Farbstoffe üblichen Färbe- und Druckverfahren Färbungen und Drucke in kräftigen, marineblauen, echten Tönen.

Beispiel 69

Zu einer neutralen Lösung von 0°C von 53 Teilen der Amino-Azoverbindung 4-(2'-Sulfo-5'-amino-phenyl]-azo-3-methyl-1-(4''-β-sulfatoethylsulfonyl-phenyl)-pyrazol-5-on in 300 Teilen Wasser gibt man 15 Teile Trifluortriazin und führt die Umsetzung bei einem pH-Wert zwischen 4 und 5 und einer Temperatur zwischen 0 und 10°C durch. Danach gibt man 39 Teile Bis-[γ-(β'-chlorethylsulfonyl)-propyl]-amin-Hydrochlorid hinzu und führt die zweite Kondensationsreaktion bei einem pH-Wert von 8 durch.

Der erfindungsgemäße Azofarbstoff wird in üblicher Weise isoliert, beispielsweise durch Aussalzen mit Natriumchlorid. Er besitzt, in Form der freien Säure geschrieben, die Formel

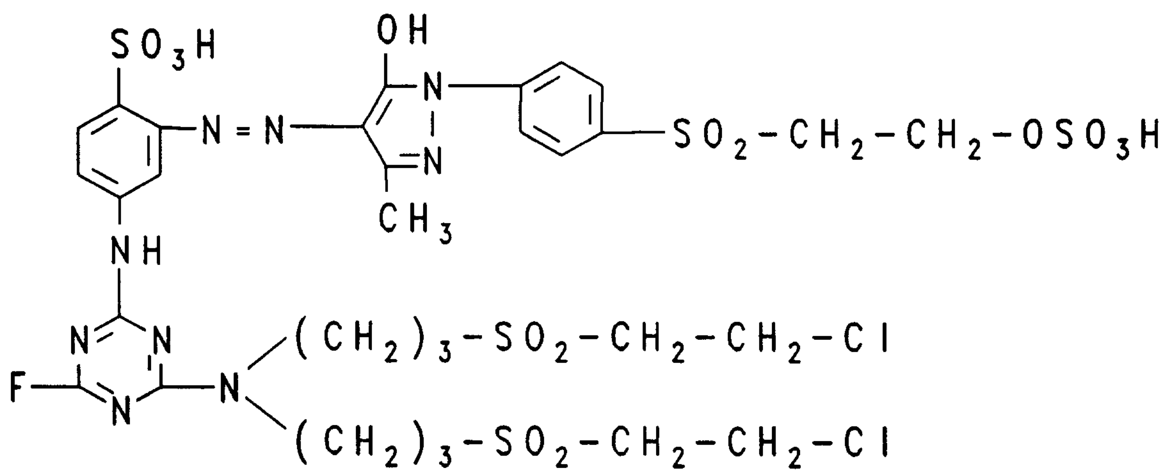

und liefert beispielsweise auf Baumwolle nach den üblichen Anwendungsverfahren farbstarke, gelbe Färbungen und Drucke mit guten Echtheitseigenschaften.

Beispiel 70

Zur Herstellung eines erfindungsgemäßen Azofarbstoffes verfährt man gemäß der Verfahrensweise des Beispieles 69, geht jedoch von der Amino-Azoverbindung 4-(2'-Sulfo-4'-amino-phenyl)-azo-3-methyl-1-(4''-β-sulfatoethylsulfonyl-phenyl]-pyrazol-5-on aus.
Man erhält den erfindungsgemäßen Azofarbstoff der Formel (in Form der freien Säure geschrieben)

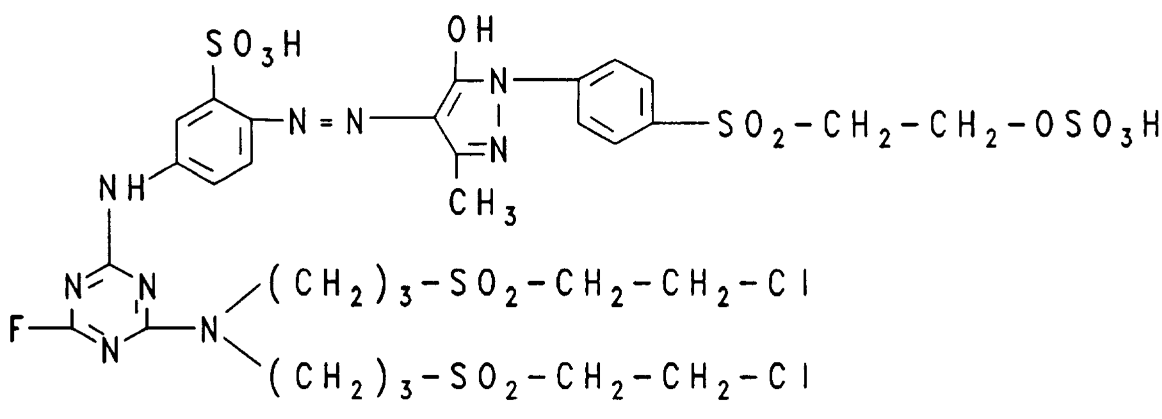

der beispielsweise Baumwolle in farbstarken gelben Tönen mit guten Echtheitseigenschaften färbt.

Beispiel 71

Eine neutrale Lösung von 63 Teilen der Amino-disazo-Ausgangsverbindung 4-[4'-(4''-β-Sulfatoethylsulfonyl-phenyl)-azo-7'-sulfo-naphth-1'-yl]-azo-3-methyl-anilin in 1000 Teilen Wasser setzt man bei 0°C unter

Einhaltung eines pH-Wertes von 4 bis 5 mittels 15%iger wäßriger Natriumcarbonatlösung mit 15 Teilen Trifluortriazin um. Unter weiterem Rühren gibt man anschließend 39 Teile Bis-[γ-(β'-chlorethylsulfonyl)-propyl]-amin-Hydrochlorid hinzu und führt die Umsetzung bei 10 °C und einem pH-Wert von 8 zu Ende.

Man isoliert den erfindungsgemäßen Disazofarbstoff der Formel (in Form der freien Säure geschrieben)

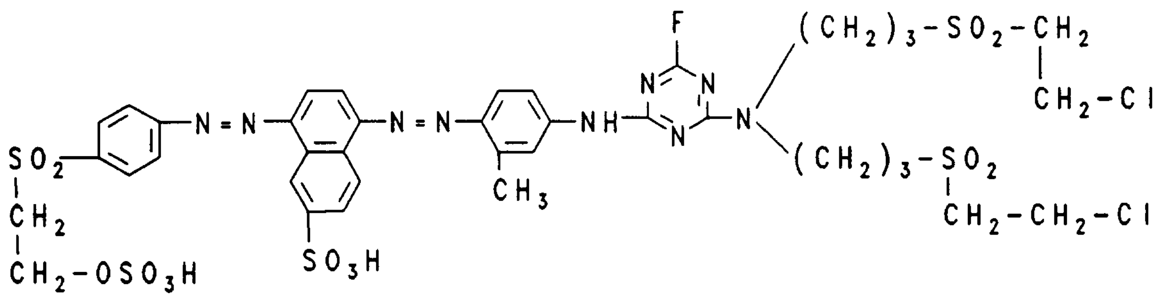

aus der Syntheselösung bei einem pH-Wert von 7 durch Aussalzen mit Natriumchlorid als Alkalimetallsalz (Natriumsalz). Er zeigt sehr gute anwendungstechnische Eigenschaften und färbt beispielsweise Baumwolle nach den in der Technik üblichen Färbe- und Druckverfahren in farbstarken braunen Tönen mit guten Echtheitseigenschaften.

Beispiel 72

Zur Herstellung eines erfindungsgemäßen Disazofarbstoffes verfährt man gemäß der Verfahrensweise des Beispieles 71, geht jedoch von der Ausgangsverbindung 4-{4'-[2''-Sulfo-4''-(β-sulfatoethylsulfonyl)-phenyl]-azo-7'-sulfo-naphth-1'-yl}-azo-3-methyl-anilin aus. Man erhält den erfindungsgemäßen Disazofarbstoff der Formel (in Form der freien Säure geschrieben)

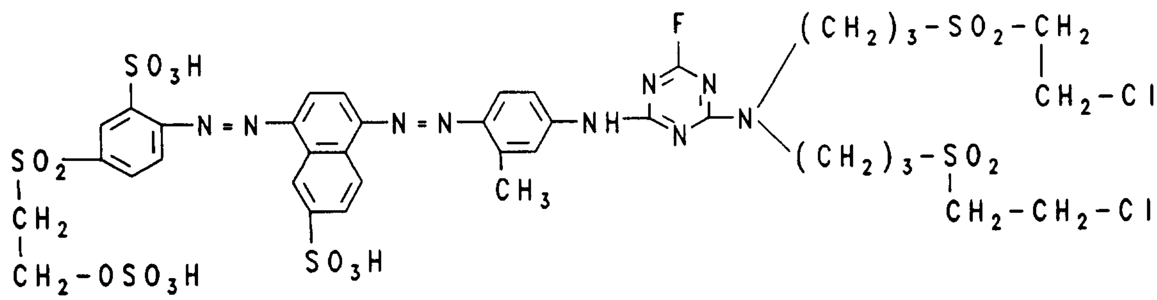

der beispielsweise Baumwolle nach den in der Technik üblichen Anwendungsverfahren in farbstarken, braunen, echten Tönen färbt.

Beispiel 73

Zur Herstellung einer erfindungsgemäßen Disazoverbindung verfährt man analog den Angaben des Beispieles 69, führt die Umsetzung jedoch anstelle mit Trifluortriazin mit der äquivalenten Menge an Trichlortriazin durch. Man erhält den analogen Chlortriazinfarbstoff, der beispielsweise Baumwolle nach den in der Technik für faserreaktive Farbstoffe üblichen Färbe- und Druckverfahren in kräftigen gelben Tönen mit guten Echtheiten färbt.

Beispiel 74

Zur Herstellung eines erfindungsgemäßen Monoazofarbstoffes verfährt man analog der Verfahrensweise des Beispieles 69, geht jedoch von der Amino-azo-Ausgangsverbindung 4-(2'-Sulfo-4'-amino-phenyl)-azo-3-methyl-1-(4''-β-sulfatoethylsulfonyl-phenyl)-pyrazol-5-on und anstelle, von Trifluortriazin, von Trichlortriazin in den entsprechenden äquivalenten Mengen aus. Man erhält den erfindungsgemäßen Azofarbstoff der

Formel

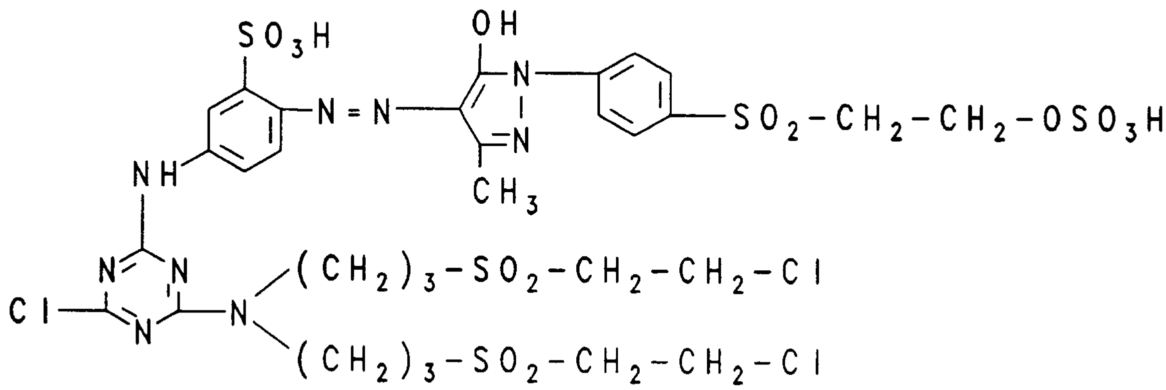

der beispielsweise Baumwolle in farbstarken, gelben Tönen mit guten Echtheitseigenschaften färbt.

Beispiel 75

Zur Herstellung eines erfindungsgemäßen Azofarbstoffes verfährt man analog den Angaben des Beispieles 71, setzt jedoch anstelle von Trifluortriazin die äquivalente Menge an Trichlortriazin ein. Man erhält den entsprechenden Chlortriazinfarbstoff, der die in der Beschreibung genannten Fasermaterialien, wie insbesondere Cellulosefasermaterialien, nach den in der Technik für faserreaktive Farbstoffe üblichen Anwendungsverfahren in farbstarken braunen, echten Tönen färbt.

Beispiel 76

Zur Herstellung eines erfindungsgemäßen Azofarbstoffes setzt man analog den Angaben des Beispieles 72 die dort genannte Amino-disazo-Ausgangsverbindung mit Trichlortriazin und anschließend mit Bis-[γ-(β'-chlorethylsulfonyl)-propyl]-amin-Hydrochlorid um und erhält den analogen erfindungsgemäßen Chlortriazinfarbstoff, der Baumwolle in farbstarken, echten, braunen Tönen färbt.

**Patentansprüche**

**1.** Azofarbstoff der allgemeinen Formel (1)

$(Y^o - SO_2)_a - F - Z$ (1)

in welcher bedeuten:

F ist der Rest eines sulfogruppenhaltigen Mono-, Dis- oder Polyazofarbstoffes, wie Trisazofarbstoffes;

$Y^o$ ist Vinyl oder ist Ethyl, das in β-Stellung durch einen unter Einwirkung von Alkali bei Bildung der Vinylgruppe eliminierbaren Substituenten substituiert ist;

Z ist eine Gruppe der allgemeinen Formel (2)

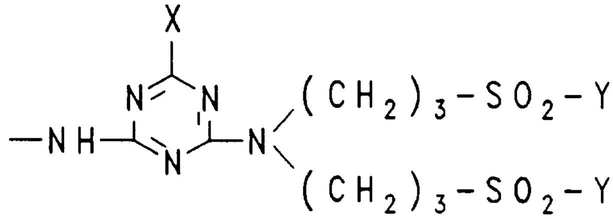

(2)

in welcher

X Chlor oder Fluor ist und

Y Vinyl ist oder Ethyl bedeutet, das in $\beta$-Stellung durch einen unter Einwirkung von Alkali bei Bildung der Vinylgruppe eliminierbaren Substituenten substituiert ist;

a ist die Zahl 1 oder 2.

**2.** Azofarbstoff nach Anspruch 1 entsprechend der allgemeinen Formel (1a) oder (1b)

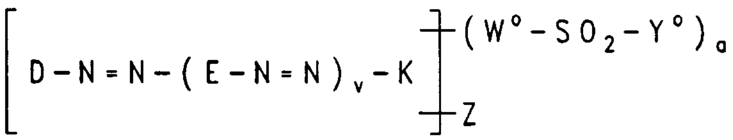

(1a)

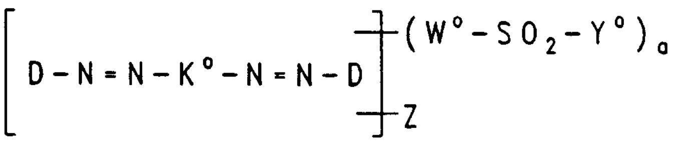

(1b)

in welcher bedeuten:

D ist als Rest einer Diazokomponente ein gegebenenfalls durch übliche Substituenten substituierter Benzol- oder Naphthalinring, wobei D auch einen gegebenenfalls substituierten Phenylazo- oder Naphthylazo-Rest besitzen kann;

E ist als Rest einer diazotierbaren Kupplungskomponente ein gegebenenfalls durch übliche Substituenten substituierter Benzol- oder Naphthalinring, der in ortho-Stellung zur Azogruppe eine Hydroxy- oder Aminogruppe besitzen kann;

K ist der Rest einer in Azofarbstoffen üblichen Kupplungskomponente;

v ist die Zahl Null oder 1;

$K^o$ ist der Rest einer bivalenten Kupplungskomponente;

$W^o$ ist eine Alkylengruppe von 1 bis 4 C-Atomen oder eine Gruppe der allgemeinen Formel -NH-($C_2$-$C_4$-alkylen)- oder ist eine kovalente Bindung;

$Y^o$, a und Z haben eine der in Anspruch 1 genannten Bedeutungen;

in Formel (1a) stehen die Gruppe(n) $-W^o-SO_2-Y^o$ an D und die Gruppe Z an K oder die Gruppe Z an D und die Gruppe(n) $-W^o-SO_2-Y^o$ an K gebunden, und

in Formel (1b) stehen die Gruppe(n) $-W^o-SO_2-Y^o$ an das eine D und die Gruppe Z an das andere D gebunden.

**3.** Mono-, Dis- und Trisazofarbstoff nach Anspruch 1 entsprechend der allgemeinen Formeln (1c), (1d) oder (1e)

$$R^G - D^1 - N = N - (E - N = N)_v - K^2 - Z \quad (1c)$$

$$Z - D^2 - N = N - (E - N = N)_v - K^1 - R^G \quad (1d)$$

$$R^G - D^1 - N = N - K^o - N = N - D^2 - Z \quad (1e)$$

in welchen bedeuten:

$R^G$-$D^1$- ist ein Rest der nachstehend angegebenen und definierten allgemeinen Formel (3a) oder (3b);

Z-$D^2$- ist ein Rest der nachstehend angegebenen und definierten allgemeinen Formel (4a) oder (4b);

E ist ein Rest der nachstehend angegebenen und definierten allgemeinen Formel (5a), (5b) oder (5c);

v ist die Zahl Null oder 1;

-$K^1$-$R^G$ ist eine Gruppe der nachstehend angegebenen und definierten allgemeinen Formel (6a), (6b), (6c), (6d), (6e), (6f), (6g) oder (6h);

-$K^2$-Z ist eine Gruppe der nachstehend angegebenen und definierten allgemeinen Formel (7a),

(7b), (7c), (7d), (7e), (7f), (7g), (7h) oder (7i);

K° ist der 1-Amino-8-hydroxy-3,6- oder -4,6-disulfo-naphth-2,7-ylen-Rest

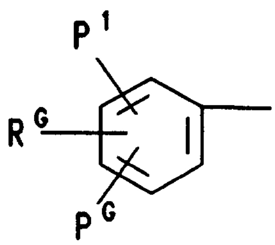

(3a)

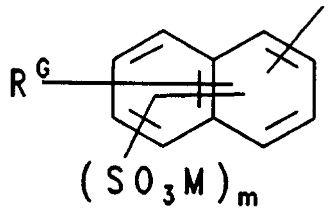

(3b)

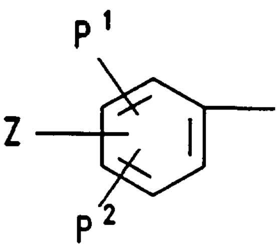

(4a)

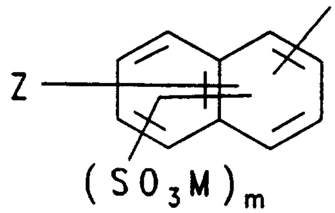

(4b)

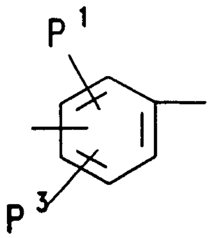
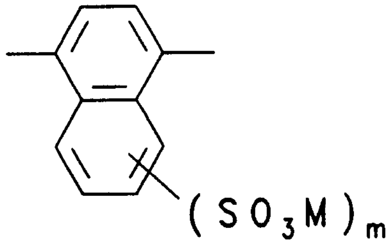
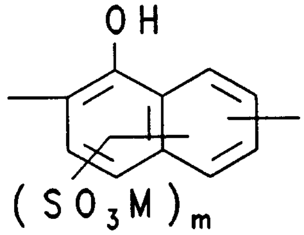

(5a) (5b) (5c)

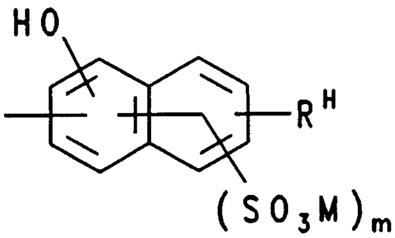
(6a)

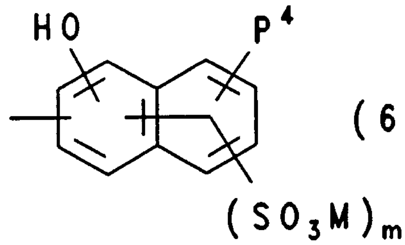
(6b)

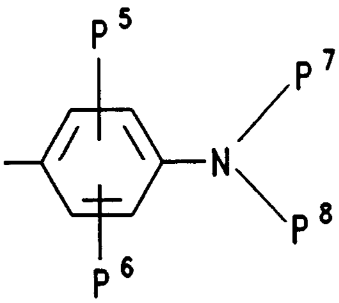
(6c)

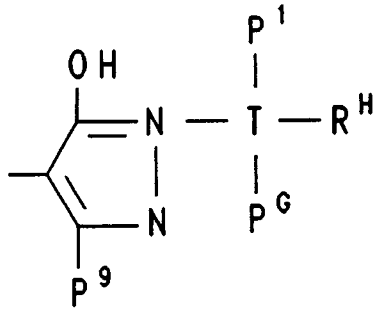
(6d)

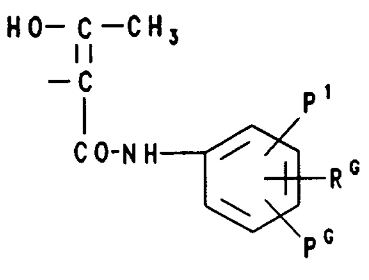
(6e)

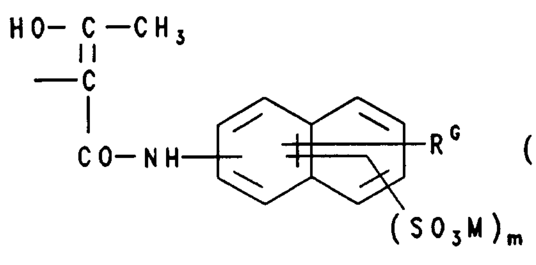
(6f)

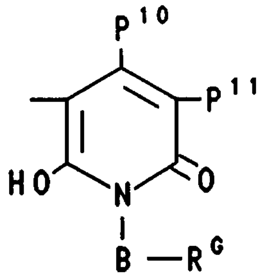

(6g)

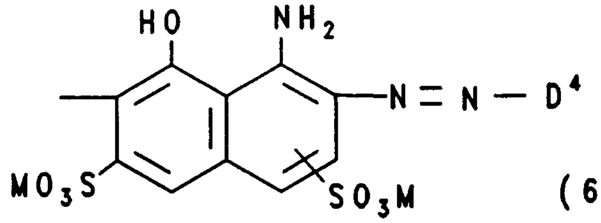

(6h)

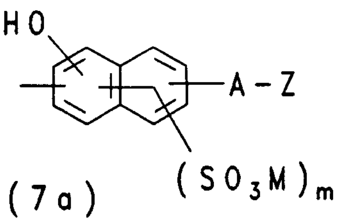

(7a)

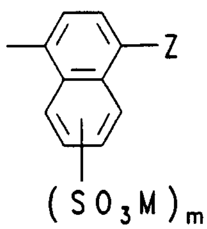

(7b)

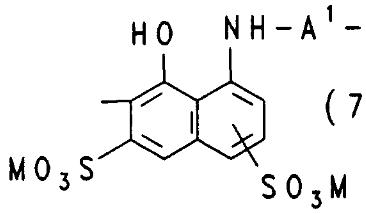

(7c)

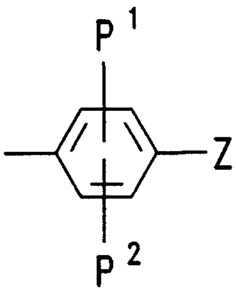

(7d)

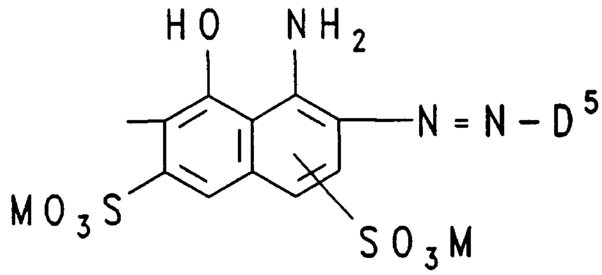

(7e)

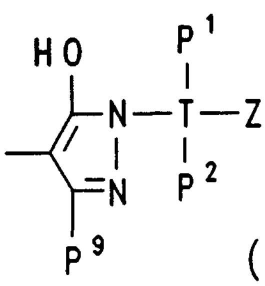

(7f)

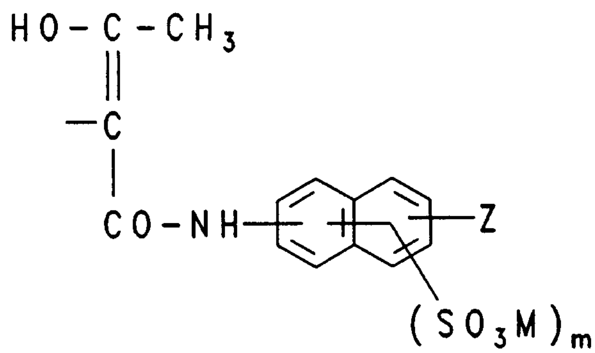

(7g)

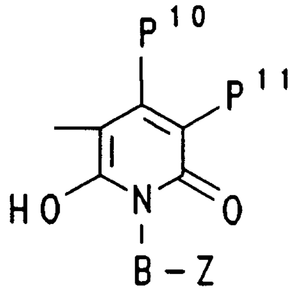

(7h)

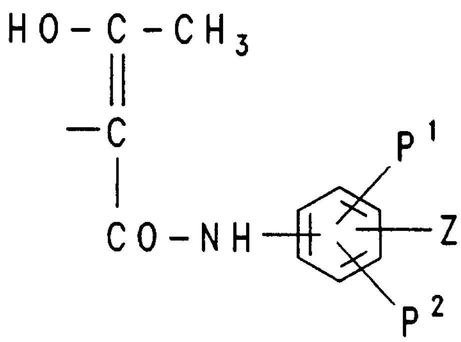

(7i)

in welchen bedeuten:

M ist Wasserstoff oder ein Alkalimetall;

Z hat eine der in Anspruch 1 genannten Bedeutungen;

$Z^1$ ist ein Rest der allgemeinen Formel (2A)

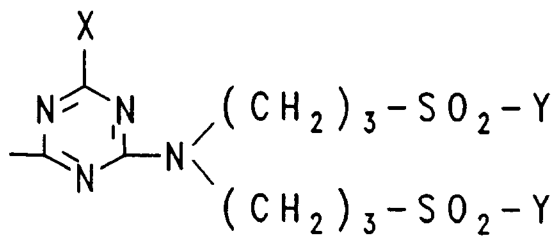

(2A)

in welcher X und Y die in Anspruch 1 genannten Bedeutungen haben;

$R^G$ ist eine Gruppe der allgemeinen Formel $Y^o$-$SO_2$-$W^o$-, in welcher $Y^o$ die in Anspruch 1 genannte Bedeutung hat und $W^o$ eine Alkylengruppe von 1 bis 4 C-Atomen oder eine Gruppe der allgemeinen Formel -($C_2$-$C_4$-alkylen)-NH- oder eine kovalente Bindung ist, oder ist eine Gruppe der allgemeinen Formel $R^G$-$D^3$-N = N-, in welcher $R^G$ hier einen Rest der oben definierten allgemeinen Formel $Y^o$-$SO_2$-$W^o$- bedeutet und $D^3$ einen Phenylenrest darstellt, der die später definierten Substituenten $P^1$ und $P^G$ enthält, oder einen Naphthylenrest bedeutet, der durch 1 oder 2 Sulfogruppen substituiert ist;

$P^1$ ist Wasserstoff, Methyl, Ethyl, Methoxy, Ethoxy, Hydroxy, Alkanoyl von 2 bis 5 C-Atomen, Cyano, Sulfo, Carboxy, Nitro, Phenylamino, Phenoxy, Sulfophenylamino, Alkoxycarbonyl von 2 bis 5 C-Atomen Sulfamoyl, Carbamoyl, N-($C_1$-$C_4$-Alkyl)-carbamoyl, Fluor, Chlor, Brom oder Trifluormethyl;

$P^2$ ist Wasserstoff, Methyl, Ethyl, Methoxy, Ethoxy, Cyano, Carboxy, Sulfo, Chlor, Alkanoylamino von 2 bis 5 C-Atomen, Alkoxycarbonyl von 2 bis 5 C-Atomen, Carbamoyl, N-($C_1$-$C_4$-Alkyl)-sulfamoyl, N-Sulfophenyl- amidocarbonyl, N-Phenyl-amidocarbonyl, Alkylsulfonyl von 1 bis 4 C-Atomen, Phenylsulfonyl oder Phenoxy;

$P^G$ ist Wasserstoff, Methyl, Ethyl, Methoxy, Ethoxy, Cyano, Carboxy, Sulfo, Chlor, Alkanoylamino von 2 bis 5 C-Atomen, Alkoxycarbonyl von 2 bis 5 C-Atomen, Carbamoyl, N-($C_1$-$C_4$-Alkyl)-sulfamoyl, N-Sulfophenyl- amidocarbonyl, N-Phenyl-amidocarbonyl, Alkylsulfonyl von 1 bis 4 C-Atomen, Phenylsulfonyl oder Phenoxy oder ist eine Gruppe der oben definierten allgemeinen Formel

$Y^o$-$SO_2$-$W^o$-;

m steht für die Zahl Null, 1 oder 2 (wobei diese Gruppe im Falle von m gleich Null ein Wasserstoffatom bedeutet);

$P^3$ ist Wasserstoff, Alkyl von 1 bis 4 C-Atomen, Alkoxy von 1 bis 4 C-Atomen, Chlor, Amino, Alkanoylamino von 2 bis 5 C-Atomen, Benzoylamino, Ureido, Phenylureido, Alkylureido mit 1 bis 4 C-Atomen im Alkylrest, Phenylsulfonyl oder Alkylsulfonyl von 1 bis 4 C-Atomen;

$R^H$ ist eine Gruppe der oben definierten allgemeinen Formel -$W^o$-$SO_2$-$Y^o$ oder eine Gruppe der

allgemeinen Formel -N = N-$K^3$, in welcher $K^3$ eine Gruppe der oben angegebenen allgemeinen Formel (6c), (6d), (6e) oder (6h) ist, in welchen $R^H$ jedoch hier eine Gruppe der oben definierten allgemeinen Formel -$W^o$-$SO_2$-$Y^o$ ist;

$P^4$ ist Phenylureido, das im Phenylrest durch Substituenten aus der Gruppe Chlor, Methyl, Methoxy, Sulfo und Carboxy substituiert sein kann und durch eine Gruppe der oben definierten allgemeinen Formel -$W^o$-$SO_2$-$Y^o$ substituiert ist, oder ist Alkanoylamino von 2 bis 5 C-Atomen oder ist Benzoylamino, das im Benzolrest durch Substituenten aus der Gruppe Chlor, Methyl, Methoxy, Nitro, Sulfo und Carboxy substituiert sein kann und durch eine Gruppe der oben definierten allgemeinen Formel -$W^o$-$SO_2$-$Y^o$ substituiert ist;

$P^5$ ist Wasserstoff, Alkyl von 1 bis 4 C-Atomen, Alkoxy von 1 bis 4 C-Atomen, Brom, Chlor oder Alkanoylamino von 2 bis 7 C-Atomen;

$P^6$ ist Alkyl von 1 bis 4 C-Atomen Alkoxy von 1 bis 4 C-Atomen, Chlor, Alkanoylamino von 2 bis 7 C-Atomen, Ureido oder Phenylureido;

$P^7$ ist Wasserstofff oder Alkyl von 1 bis 4 C-Atomen, das durch Hydroxy, Cyano, Carboxy, Sulfo, Sulfato, Methoxycarbonyl, Ethoxycarbonyl oder Acetoxy oder eine Gruppe der oben definierten allgemeinen Formel -$SO_2$-$Y^o$ substituiert sein kann;

$P^8$ ist Alkyl von 1 bis 4 C-Atomen, das durch Hydroxy, Cyano, Carboxy, Sulfo, Sulfato, Methoxycarbonyl, Ethoxycarbonyl oder Acetoxy oder eine Gruppe der oben definierten allgemeinen Formel -$SO_2$-$Y^o$ substituiert sein kann, oder ist Benzyl oder Phenyl oder durch Alkyl von 1 bis 4 C-Atomen, Alkoxy von 1 bis 4 C-Atomen, Chlor und/oder Sulfo substituiertes Benzyl oder Phenyl, wobei diese Phenylreste auch durch eine Gruppe der oben definierten allgemeinen Formel -$W^o$-$SO_2$-$Y^o$ substituiert sein können und wobei mindestens einer der Reste $P^7$ und $P^8$ einen Rest der allgemeinen Formel -$SO_2$-$Y^o$ enthält;

$P^9$ ist Wasserstoff, Alkyl von 1 bis 4 C-Atomen, Cyano, Carboxy, Carbalkoxy von 2 bis 5 C-Atomen, Carbamoyl oder Phenyl;

T steht für einen Benzol- oder Naphthalinring;

$P^{10}$ ist Wasserstoff oder Alkyl von 1 bis 4 C-Atomen oder durch Alkoxy von 1 bis 4 C-Atomen oder Cyano substituiertes Alkyl von 1 bis 4 C-Atomen oder ist Phenyl;

$P^{11}$ ist Wasserstoff, Chlor, Brom, Sulfo, Carbamoyl, Methylsulfonyl, Phenylsulfonyl, Cyano oder Sulfoalkyl von 1 bis 4 C-Atomen;

A ist ein Rest der Formel -NH-phenylen- oder -NH-CO-phenylen- oder -NH-CO-NH-phenylen- oder eine kovalente Bindung;

$A^1$ ist ein Rest der Formel -phenylen-NH- oder -CO-phenylen-NH- oder -CO-NH-phenylen-NH- oder eine kovalente Bindung;

B ist Alkylen von 1 bis 4 C-Atomen, das durch Hydroxy, Sulfato, Sulfo, Carboxy, Phosphato oder Acetyloxy substituiert sein kann, oder ist Methylenphenylen, Ethylenphenylen, Phenylenmethylen, Phenylenethylen oder Phenylen oder im Benzolrest durch Fluor, Chlor, Brom, Methyl, Methoxy, Cyano, Sulfo, Carboxy, Acetyl, Nitro, Carbamoyl und/oder Sulfamoyl substituiertes Methylenphenylen, Ethylenphenylen oder Phenylen;

$D^4$ ist ein Rest der oben angegebenen allgemeinen Formel (3a) oder (3b), in welchen $P^1$ und $P^2$ eine der obengenannten Bedeutungen besitzt und $R^G$ hier eine Gruppe der oben definierten allgemeinen Formel -$W^o$-$SO_2$-$Y^o$ ist;

$D^5$ ist eine Gruppe der oben genannten und definierten allgemeinen Formel (4a) oder (4b);

in den Formeln (6a), (6b) und (7a) steht die freie Bindung, die zur Azogruppe führt, in ortho-Stellung zur Hydroxygruppe an den aromatischen Kern gebunden, und in Formel (5c) steht die freie Bindung, die zm Rest -N = N-$K^1$-$R^G$ oder -N = N-$K^2$-Z führt, in 2- oder 3-Stellung an den 8-Hydroxy-naphthyl-Rest gebunden, und in den Formeln (6a) und (6f) steht $R^H$, falls $R^H$ eine Gruppe -N = N-$K^3$ bedeutet, sowie in Formel (7a) der Rest Z in $\beta$-Stellung des jeweiligen Naphthalinrestes gebunden.

**4.** Verfahren zur Herstellung der Azofarbstoffe der allgemeinen Formel (1) von Anspruch 1, dadurch gekennzeichnet, daß man für den jeweiligen Farbstoff typische Vorprodukte, von denen mindestens eines eine Gruppe der Formel $Y^o$-$SO_2$- und mindestens eines eine Gruppe der allgemeinen Formel (2) enthält, miteinander umsetzt, oder daß man eine Halogen-triazin-Verbindung der allgemeinen Formel (21)

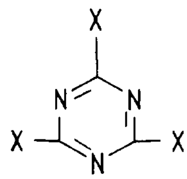

(21)

in welcher X eine der in Anspruch 1 genannten Bedeutungen besitzt, mit einer aminogruppenhaltigen Ausgangsverbindung der allgemeinen Formel (20a) oder (20b)

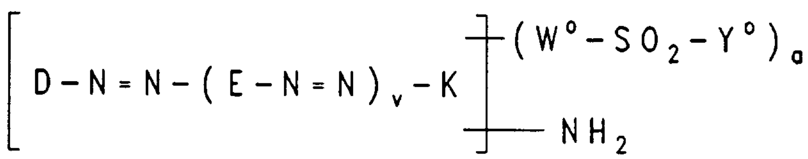

(20a)

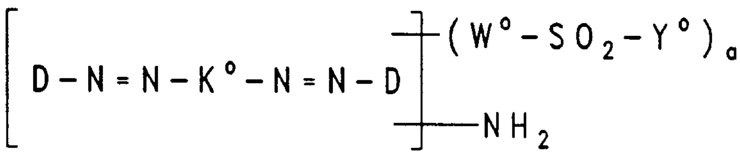

(20b)

in welchen D, E, K, K°, v, W°, Y° und a die in Anspruch 1, 2 oder 3 genannten Bedeutungen haben, wobei in Formel (20a) die Gruppe(n) $-W^o-SO_2-Y^o$ an D und die Aminogruppe $-NH_2$ an K oder die Aminogruppe an D und die Gruppe(n) $-W^o-SO_2-$ an K gebunden und in Formel (20b) die Gruppe(n) $-W^o-SO_2-Y^o$ an das eine D und die Aminogruppe an das andere D gebunden sind,
und mit einer Aminoverbindung der allgemeinen Formel (22)

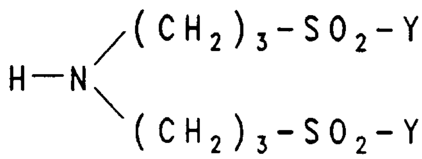

(22)

in welcher Y die in Anspruch 1 genannte Bedeutung hat, in beliebiger Reihenfolge miteinander umsetzt.

**5.** Verwendung eines Farbstoffes von Anspruch 1 oder eines nach Anspruch 4 hergestellten Farbstoffes zum Färben (einschließlich Bedrucken) von hydroxy- und/oder carbonamidgruppenhaltigem Material, insbesondere Fasermaterial.

**6.** Verfahren zum Färben (einschließlich Bedrucken) von hydroxy- und/oder carbonamidgruppenhaltigem Material, insbesondere Fasermaterial, bei welchem man einen Farbstoff auf das Material aufbringt und den Farbstoff auf dem Material mittels Wärme oder mit Hilfe eines alkalisch wirkenden Mittels oder mittels beider Maßnahmen fixiert, dadurch gekennzeichnet, daß man als Farbstoff einen Farbstoff von Anspruch 1 oder einen nach Anspruch 4 hergestellten Farbstoff einsetzt.

Europäisches Patentamt

# EUROPÄISCHER RECHERCHENBERICHT

Nummer der Anmeldung
EP 94 10 9158

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| X | FR-A-2 346 418 (CASSELLA FARBWERKE MAINKUR AG)<br>* Seite 1, Zeile 20 - Zeile 21 *<br>* Seite 7, Zeile 35 * | 1,2,4-6 | C09B62/08<br>C09B62/507 |
| D | & GB-A-1 576 297 (...)<br>--- | | |
| D,A | EP-A-0 070 808 (CIBA-GEIGY AG)<br>* Seite 13, Zeile 11 *<br>* Seite 13, Zeile 15 - Zeile 24; Beispiele 157,165 *<br>--- | 1-6 | |
| D,A | EP-A-0 374 758 (HOECHST AG)<br>* Seite 8, Zeile 32 - Seite 10, Zeile 42 *<br>----- | 1-6 | |
| | | | RECHERCHIERTE SACHGEBIETE (Int.Cl.5) |
| | | | C09B |

Der vorliegende Recherchenbericht wurde für alle Patentansprüche erstellt

| Recherchenort | Abschlußdatum der Recherche | Prüfer |
|---|---|---|
| DEN HAAG | 22. September 1994 | Ketterer, M |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)